# EUROPEAN PATENT SPECIFICATION

(11) **EP 1 181 274 B1**
(45) Date of publication and mention of the grant of the patent: **10.11.2010**
(21) Application number: 00930662.2
(22) Date of filing: 11.05.2000
(51) Int. Cl.: C07D 207/12, A61K 31/40, A61P 19/02

(54) **IMINOCYCLITOL INHIBITORS OF HEXOAMINIDASE AND GLYCOSIDASE**
IMINOCYCLITOL-INHIBITOREN VON HEXOAMINIDASE UND GLYCOSIDASE
IMINOCYCLITOLS INHIBITEURS DE L'HEXOAMINIDASE ET DE LA GLYCOSIDASE

(30) Priority: 11.05.1999 US 133549 P
(43) Date of publication of application: 27.02.2002
(73) Proprietor: THE SCRIPPS RESEARCH INSTITUTE, La Jolla, CA 92037 (US)
(72) Inventor: WONG, Chi-Huey, Rancho Santa Fe, CA 92067 (US); LIU, Jungie, San Diego, CA 92117 (US)
(74) Representative: Hedley, Nicholas James Matthew
(86) International application number: PCT/US2000/013048
(87) International publication number: WO 2000/068194

(56) References cited:
- US-A- 5 250 703
- US-A- 5 451 679
- TAKEBAYASHI, MAKI ET AL: "A Versatile Synthetic Strategy for the Preparation and Discovery of New Iminocyclitols as Inhibitors of Glycosidases" JOURNAL OF ORGANIC CHEMISTRY (1999), 64(14), 5280-5291, 24 June 1999 (1999-06-24), XP002264343
- BUTTERS, T. D. ET AL: "Molecular requirements of imino sugars for the selective control of N-linked glycosylation and glycosphingolipid biosynthesis" TETRAHEDRON: ASYMMETRY , 11(1), 113-124 CODEN: TASYE3; ISSN: 0957-4166, 2000, XP002331374
- QIAN, XINHUA ET AL: "C2-symmetrical tetrahydroxyazepanes as inhibitors of glycosidases and HIV/FIV proteases" BIOORGANIC & MEDICINAL CHEMISTRY (1996), 4(12), 2055-2069, 1996, XP002264344
- HIRANUMA, SAYOKO ET AL: "Synthesis and inhibition analysis of five-membered homoaza sugars from D-arabinofuranose via an SN2 reaction of the chloromethylsulfonate" TETRAHEDRON LETTERS , 36(45), 8247-50 CODEN: TELEAY; ISSN: 0040-4039, 1995, XP002331375
- TAKAOKA ET AL.: 'Inhibition of N-acetylglucosaminyltransfer enzymes: chemical-enzymatic synthesis of new five-membered acetamido azasugars' J. ORG. CHEM. vol. 58, no. 18, 1993, pages 4809 - 4815, XP002930900

## Description

### Technical Field:

The present invention relates to the inhibition of hexoaminidase and glycosidase. More particularly, the present invention relates to the selective inhibition of hexoaminidases and glycosidases using designed iminocylitols.

### Background:

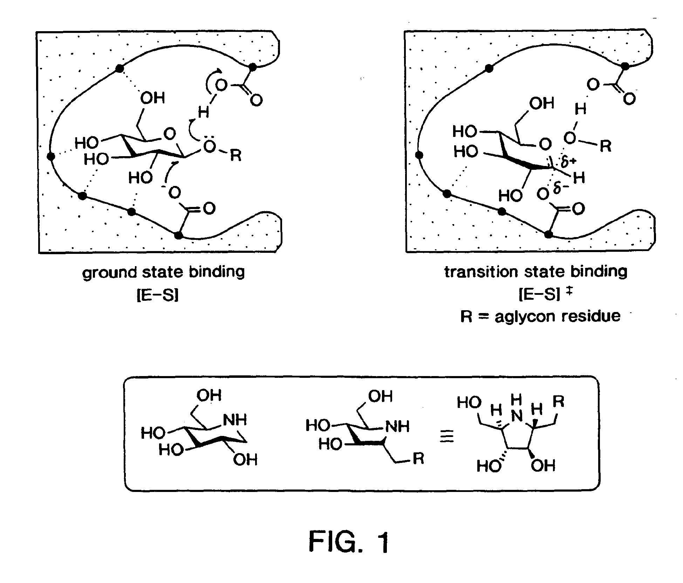

Enzymatic hydrolysis of glycosidic bonds generally takes place *via* general acid-base catalyses that require two critical residues, a proton donor and a nucleophile. The process is illustrated in Figure 1. Five- or six membered iminocyclitols carrying hydroxyl groups with specific orientation and a secondary amine have been used to mimic the shape and charge of the transition state of the reaction and have been shown to be potent inhibitors of such enzymes (T.A. Beyer, et al., J. Biol. Chem. 1979, 254, 12531-12541; H. Paulsen, et al., Adv. Carbohydr. Chem. Biochem. 1968, 23, 115-232; A.B. Hughes, et al., J. Nat. Prod. Rep. 1994, 135-162; C.-H. Wong, et al., Angew. Chem., Int. Ed. Engl., 1995, 34, 412-432 and 521-546; B. Ganem, Acc. Chem. Res. 1996, 29, 340-347; S. Picasso, Chimia, 1996, 50, 648-649; L.A.G.M. van den Broek, in Carbohydr. Drug Des. 1997, Eds by Z.J. Witczak, et al., Dekker, New York, pp 1-37 and pp471-493; G.W. Fleet, et al., Tetrahedron Lett. 1985, 26, 3127-3130; Y.T Pan, et al., J. Biol. Chem. 1992, 267, 8313-8318; c) T.D. Heightman, et al., Helvetica Chim. Acta 1995, 78, 514-532; and Y. Ichikawa, et al., J. Am. Chem. Soc. 1998, 120, 3007-3018). One process for synthesizing iminocyclitols is based on aldolase-catalyzed reactions ( R.L. Pederson, et al., Tetrahedron Lett. 1988, 29, 4645-4648; T. Ziegler, et al., Angew. Chem. Int. Ed. Engl. 1988, 27, 716-717; C.H. von der Osten, et al., J. Am. Chem. Soc. 1989, 111, 2924-3927; T. Kajimoto, et al., J. Am. Chem. Soc. 1991, 113, 6187-6196; K.K.-C. Liu, et al., J. Org. Chem. 1991, 56, 6280-6289; and Y.F. Wang, et al., Angew. Chem. Int. Ed. Engl. 1994, 33, 1242-1244). Another process for synthesizing iminocyclitols is based on multi-step chemical transformations (S. Hiranuma, et al., Tetrahedron Lett. 1995, 36, 8247-8250; and C.-H. Wong, et al., J. Org. Chem. 1995, 60, 1492-1501). A preferred method for assaying inhibition activity without using radioactive isotopes employs electrospray mass spectrometry and capillary zone electrophoresis (CZE) (S. Takayama, et al., J. Am. Chem. Soc. 1997, 119, 8146-8151; J. Wu, et al., Chem. Biol. 1997, 4, 653-657; Y. Kanie, et al., Anal. Biochem. 1998, 263, 240-245; R., Zeleny, et al., Anal. Biochem. 1997, 256, 96-101; K.B. Lee, et al., Anal. Biochem. 1992, 205, 108-114; and K.-B. Lee, et al., Electrophoresis, 1991, 12, 636-640).

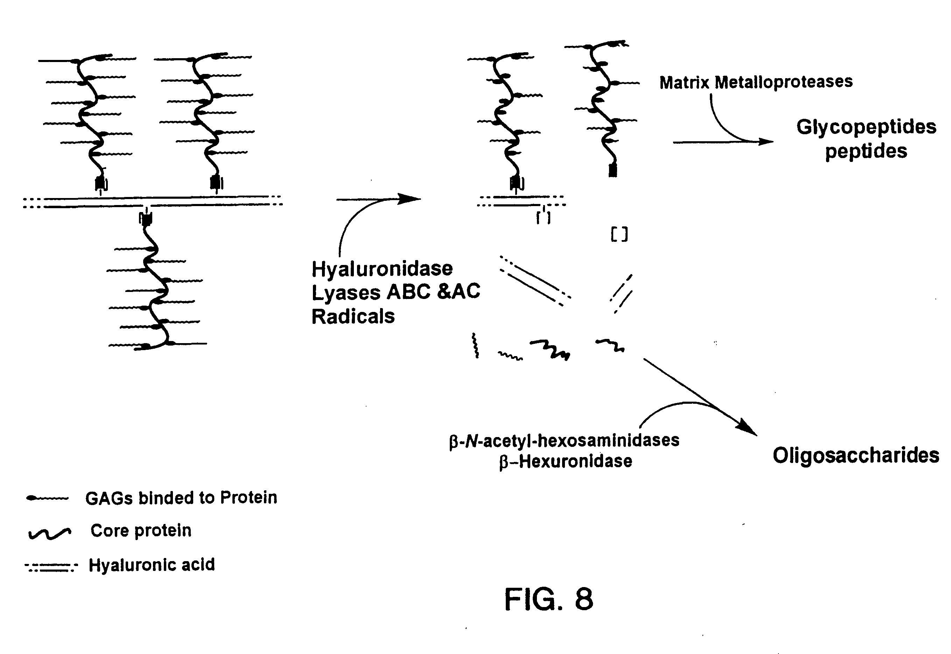

Glycosidases and hexoaminidases catalyze a myriad of clinically important processes. For example, cartilage erosion in arthritic subjects results from the over-catabolism of glycosaminoglycans (GAGs) of proteoglycan (PG)-hyaluronate complex, which fills the most part in cartilage tissue. The process is illustrated in Figure 8. The cartilage PG consists of a central protein core to which GAG side chains of chondroitin sulfate (CS) and keratan sulfate (KS) are attached together with *O*-linked and *N*-linked oligosaccharides. The PGs bind to hyaluronic acid noncovalently. The degradation of GAGs is a very complicated process, involving a multi-enzyme systems and radical reactions. It is known that subjects with arthritis have an abnormal increase of β-*N*-acetylhexoaminidases activities (O. Kida, J. Jap. Orthop. Ass. 1968, 42(6). 4010; R.W. Stephen, et al., Biochim. Biophys. Acta 1975, 399(1), 101; and J.J. Steinberg, et al., Biochim. Biophys. Acta 1983, 757(1), 47). The higher β-*N*-acetylhexoaminidases activity amplifies that of hyaluronidase and increases the degradation rate of GAG side chains.

What are needed are iminocyclitols having inhibitory activities against hexoaminidases and glycosidases.

### Summary:

One aspect of the invention is directed to designed iminocyclitols and their use for the inhibition of glycosidases. A series of five membered iminocyclitols are synthesized starting from a single starting material through Wittig reaction, Sharpless epoxidation, and double inversion reactions using the chloromethanesulfonyl group as a leaving group. This versitile synthetic strategy provides a useful route to heterocycles having activity as inhibitors of glycosidases.

It is disclosed herein that the differences in the conformation and the orientation of side chains and OH groups might be the main reason for the observed higher inhibitory activity of the 2(*R*),5(*R*)-isomer **2** compared to the 2(*S*),5(*R*)-isomer **1.** Using **2** as a starting material, a number of iminocyclitols were synthesized and tested as glycosidase inhibitors using capillary electrophoresis, and the results showed remarkable specificities toward several glycosidases. Among such compounds, **6** and **3** ∼ **5** were shown to be potent inhibitors of β-glucosidase and β-*N*-acetylglucosaminidase, respectively.

It is also disclosed herein that the amine function of compound **6** or the OH function of compound **24** may be used to make conjugates with various aglycon groups to prepare inhibitors with improved specificities. In addition, *N*-methylation of compound 3 is disclosed to enhance its inhibition activity with respect to specific enzymes.

Another aspect of the invention is directed to designed iminocyclitols and their use as inhibitors with respect to hexoaminidases, including the treatment for arthritis. Several forms of arthritis are characterized by abnormally high β-*N*-acetylhexoaminidase activity. It is disclosed herein that such forms of arthritis are treatable with designed iminocyclitols having inhibitory activity with respect to β-*N*-acetylhexoaminidase. Inhibition of β-*N*-acetylhexoaminidase activity is disclosed herein to delay and/or reduce the degradation of PG side chains and the followed cross-reactive immune response.

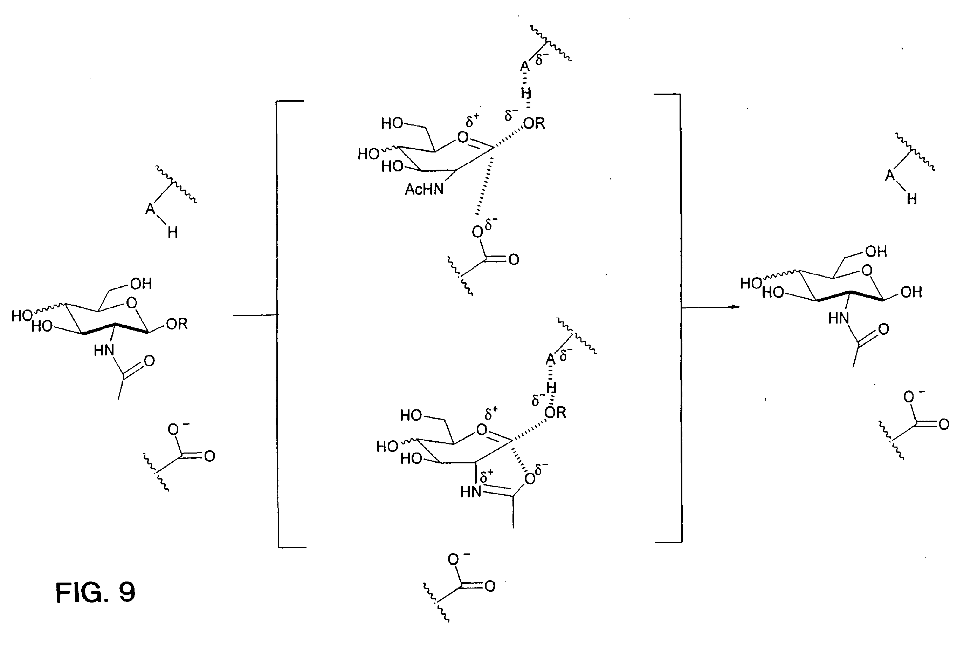

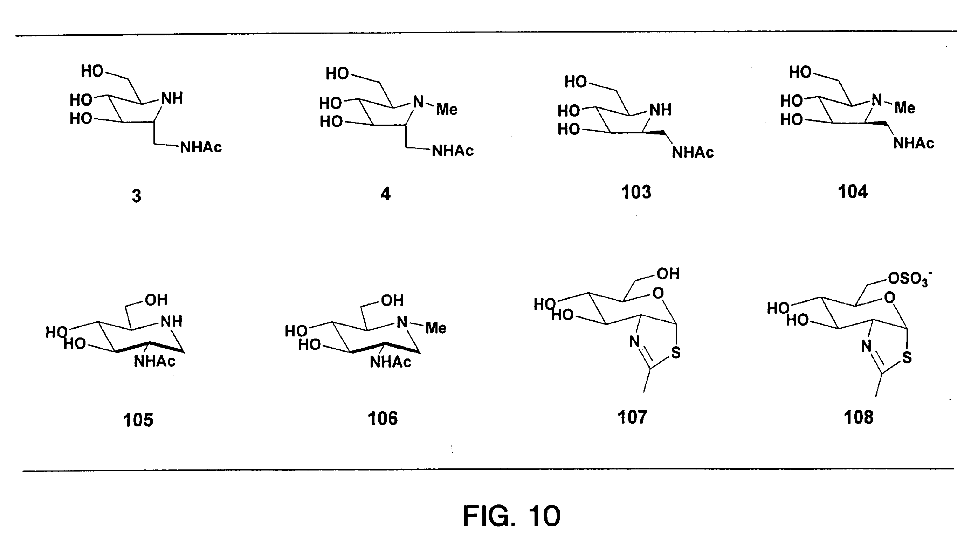

Two proposed mechanisms for the catalytic mechanism of the β-*N*-acetylhexoaminidases are illustrated in Figure 9. The first proposed mechanism employs an oxonium ion transition state (D. E. Koshland, Biol. Rev. 1953, 28, 416). According to this first mechanism, there is partial positive charge on the ring oxygen atom, which is stabilized by the depronatated carboxyl group from the enzyme. A second mechanism involving the participation of the neighboring C-2 acetamido group has also been proposed (S. Knapp, et al., J. Am. Chem. Soc. 1996, 119, 6804).
Transition states analogs corresponding to both mechanisms were synthesized and evaluated, as illustrated in Figure 10.

Because all the GAG chains except hyaluronic acid are heavily sulfated, the 6-sulfate inhibitors would mimic the molecular size and charge distribution of the mostly sulfated GAG chain substrates better than 6-OH compounds and thus should show better inhibition activity against hexoaminidases and consequentially the degradation of the GAG side chains. Accordingly, the 6-sulfate, 6-sulfate methyl ester 5-membered iminocyclitols **207, 212** and **215** were also synthesized and assayed for bioactivity.

Another aspect of the invention is directed to an inhibitor of hexoaminidase or glycosidase represented by the following structure:

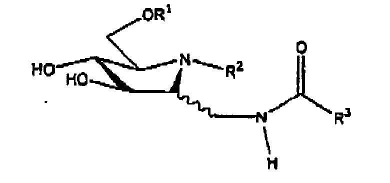

In the above structure, R¹ may be sulfate, or methyl sulfate; R² may be hydrogen, methyl, ethyl, or a branched or unbranched hydrocarbon having between 3 and 8 carbons ; and R¹ may be a hydrocarbon having between 1 and 50 carbon atoms. In a second preferred embodiment, R¹ is a sulfate group; R² is hydrogen, methyl, ethyl or any branched or unbranched hydrocarbon of between 3 and 8 carbon atoms; R³ is a hydrocarbon group that has between 1 and 20 carbon atoms. In an alternative to this second preferred embodiment, R³ is a hydrocarbon group possessing between I and 8 carbon atoms or is methyl. In a third preferred embodiment, R¹ is a methyl sulfate group; R² is selected from the group consisting of hydrogen, methyl, ethyl, and a branched or unbranched hydrocarbon of between 3 and 8 carbon atoms; and R³ is a hydrocarbon having between 1 and 20 carbon atoms. In an alternative to this third referred embodiment, R³ is a hydrocarbon having between 1 and 8 carbon atoms or is methyl.

Another aspect of the invention is directed to an inhibitor of hexoaminidase or glycosidase represented by the following structure:

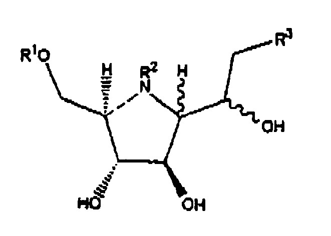

In the above structure, R¹ may be sulfate, or methyl sulfate; R² may be hydrogen, methyl, ethyl, or a branched or unbranched hydrocarbon having between 3 and 8 carbons ; and R³ may be hydroxyl or -NHC(O)R⁴, wherein R⁴ is a hydrocarbon having between 1 and 50 carbon atoms. In a second preferred embodiment, R¹ is a sulfate group; R² is hydrogen, methyl, ethyl or any branched or unbranched hydrocarbon of between 3 and 8 carbon atoms; R³ is a hydrocarbon group that has between 1 and 20 carbon atoms. In an alternative to this second preferred embodiment, R³ is a hydrocarbon group possessing between 1 and 8 carbon atoms or is methyl. In a third preferred embodiment, R¹ is a methyl sulfate group; R² is selected from the group consisting of hydrogen, methyl, ethyl, and a branched or unbranched hydrocarbon of between 3 and 8 carbon atoms; and R³ is a hydrocarbon having between 1 and 20 carbon atoms. In an alternative to this third referred embodiment, R³ is a hydrocarbon having between 1 and 8 carbon atoms or is methyl.

Another aspect of the invention is directed to a process for inhibiting the catalytic activity of a hexoaminidase or glycosidase comprising the step of contacting the hexoaminidase or glycosidase with any of the inhibitors indicated above with sufficient concentration for inhibiting the hexoaminidase or glycosidase.

Another aspect of the invention is directed to any of the inhibitors indicated above for inhibiting hexoaminidase activity within a patient, for use in treating arthritis in a subject.

### Brief Description of Drawings:

Figure 1 illustrates the proposed mechanism and transition state of (β-glucosidase-catalyzed reaction and representative structures of transition-state analogue inhibitors.

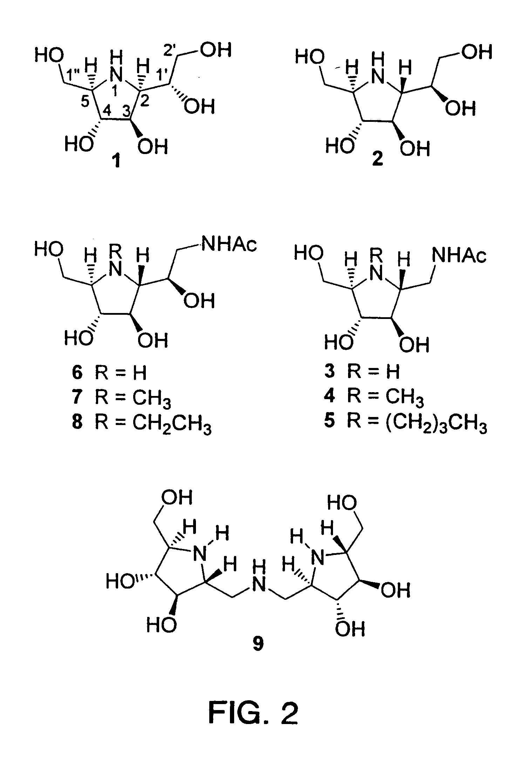

Figure 2 shows the iminocyclitol structures.
Figure 3 shows the inhibition assay results of compounds **1-9.**
Figure 4 shows the double reciprocal plots carried out to obtain Kₘ and Vₘₐₓ values. Also Kᵢ values were obtained from a replot of the slopes obtained from the double-reciprocal plot. As representative of such plots, a double-reciprocal plot of 1/ν vs 1/[S] in the β-N-acetylglucos- aminidase reaction was shown. The concentrations of 4 were (■) () nM, (□) 34 nM, (●) 67 nM, and (○) 134 nM. The Kₘ value calculated from the plot (■) was 4.1 mM. (Inset) Replot of slopes {[1/(ν-1)]/[PNP-GlcNAc]} vs **4**. Kᵢ for **4** = 0.11 µM.
Figure 5 illustrates the synthesis of compounds **1** and **2** from 2, 3, 5-benzyl-protected D-arabinofuranose **(10).**
Figure 6 illustrates the synthesis of compounds **3-8** from **21b** which was obtained by the method shown in Scheme 5.
Figure 7 illustrates the synthesis of compound **9** from compound **23.**
Figure 8 is a cartoon drawing depicting the degradation of proteoglycan-hyaluronate complex.
Figure 9 displays two proposed mechanisms of N-acetyl-β-hexosaminidases.
Figure 10 shows the inhibitors of the β-N-acetylhexosaminidases that have been investigated.
Figure 11 lists the inhibition activities of the synthesized compounds when tested with NAG-HexA.
Figure 12 shows a bar graph comparing the intracellular effects of different inhibitors.
Figure 13a graphs the different inhibition to MUG and MUGS with compound **107.**
Figure 13b graphs the different inhibition to MUG and MUGS with compound **108.**
Figure 14 illustrates the scheme used to make compounds **3, 4, 103** and **104.**
Figure 15 illustrates a second scheme used to make the 5-membered β-acetamido iminocyclitol **104.**
Figure 16 illustrates the scheme used to synthesize the 6-membered 2-acetamido-iminocyclitols **105** and **106.**
Figure 17 shows the synthesis of NAG-thiazoline **(108)** from the starting 2-acetamido-iminocyclitol **127.**
Figure 18 depicts the steps taken to synthesize the sulfated iminocyclitol **207** starting from compound **3.**
Figure 19 depicts the steps taken to synthesize the sulfated iminocyclitol **212** starting from compound **202.**
Figure 20 illustrates the two step synthesis of the sulfate methyl ester iminocyclitol **216** from compound **206.**

### Detailed Description:

### Synthesis of Glycosidase Inhibitors

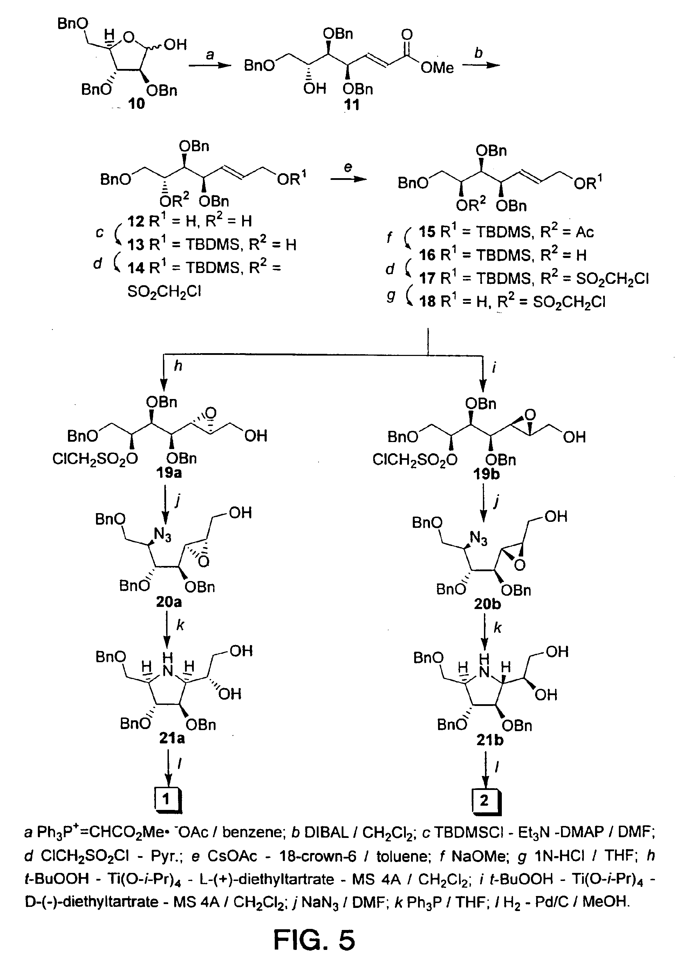

Compounds **1** and **2:**
A scheme outlining the synthesis of compounds **1** and **2** is illustrated in Figure 5. Witting reaction of 2,3,5-benzyl protected D-arabinofuranose **(10)** with methyl (triphenylphosphoranylidene)acetate afforded *E*-**11**, of which the methoxycarbonyl group was converted to the TBDMS protected alcohol (13) *via* di-*iso*-butylaluminum hydride (DIBAL) reduction followed by silylation. In order to introduce the azide function to the *R*-configuration at C-6 position, double inversion reactions were carried out. The 6-OH group was chloromesylated **(14)** and treated with CsOAc to give **15** (T. Shimizu, et al., Tetrahedron Lett. 1996, 37, 6145-6148). The leaving group was selected after several attempts using methanesulfonyl and trifluoromethanesulfonyl groups, which did not give satisfactory results. After removal of the acetate **(16),** the OH group was again chloromesylated for the second inversion **(17)** and the TBDMS group was deprotected to unmask the allylic alcohol for Sharpless epoxidation **(18).** The reason for the introduction of azide group after epoxidation is to avoid the undesired 1,3-dipole addition reaction of the introduced azide with the present double bond, which would further undergo thermolysis under the reaction conditions. The allyl alcohol **18** was epoxidized in the presence of (+)- and (-)-diethyl tartrates to afford **19a** and **19b,** respectively. No diastereoisomer was observed for either reaction according to ¹H NMR. Compounds **19a** and **19b** were treated with NaN₃ to give azides **20a** and **20b,** both of which were subjected to a reduction condition to give **21a** and **21b**. Finally, benzyl groups were hydrogenolized to give the target compounds 1 and 2, respectively. Compound **2,** while its configuration of C-1' was not determined, was isolated from Hyacintoides non-scripta (A.A. Watson, et al., J. Phytochem. 1997, 46, 255-259) and reported to have a *K*i of 4 mM against β-glucosidease from Almond and a *K*i of 85 mM against α-glucosidase from baker's yeast. Detailed comparison of ¹H and ¹³C NMR data of these compounds revealed that they were identical. It is noted that the chloromesyl group served as a very good leaving group as well as a protecting group in these transformations.

The ¹H NMR analysis of compounds **1** and **2** suggested that they adopted different ring conformations. The coupling constants for the ring protons of **1** were suggestive of a ¹*T*₂ conformation whereas **2** was adopting the ⁴*T*₃ confomation (Table 1). The detailed conformations of these compounds, however, can not be discussed only by ¹H NMR, as five member-ring compounds are known to be flexible and may exist as equilibrium mixtures (N. Asano, et al., J. Med. Chem. 1995, 38, 2349-2356; N. Asano, et al., J. Nat. Prod. 1998, 61, 625-628; and P.L. Durette, et al., Adv. Carbohydr. Chem. Biochem. 1971, 26, 49-125). It is assumed that **2** probably well mimics the transition state of the glycosidic cleavage.

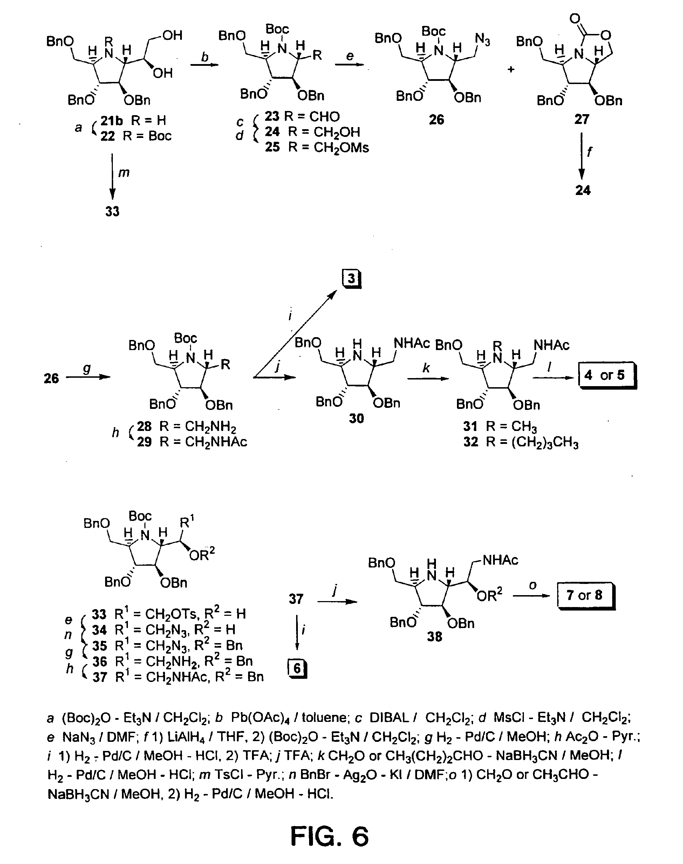

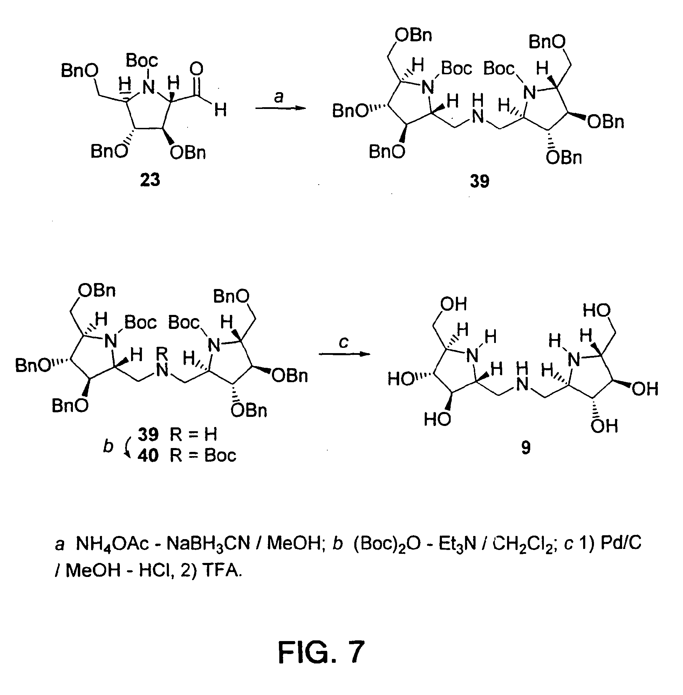

### Synthesis of compounds 3 - 9 (Figures 6 and 7)

A schematic for the synthesis of compounds **3 - 9** is illustrated in Figures 6 and 7. The 2(R),5(*R*)-configurated structures related to **2** were selected for further characterization with the following objectives:
1) to probe the critical functional groups in inhibition;
2) to introduce functional groups to the side chain for connection to an aglycon group to create a library; and
3) to examine the potency of such compounds, including those having the NHAc group as inhibitors of β-*N*-acetylglucosaminidases, as **3** is known to be a potent inhibitor of the enzyme ( Y. Takaoka, et al., J. Org. Chem. 1993, 58, 4809-4812). Synthesis of the 2(*S*)-isomer was also reported (M.H. Schumacher-Wandersleb, et al., Liebigs Ann. Chem. 1994, 555-561).
In addition to the imimocyclitol frame work with modified side chains, compounds with an alkyl group on the ring nitrogen were included because such a modification would enhance the basicity of the nitrogen atom and increase the hydrophobicity, thereby may affect the binding affinity to the target enzyme (J. Schweden, et al., Arc. Biochem. Biophys. 1986, 248, 335-340; G.W. Fleet, et al., FEBS Lett. 1988, 237, 128-132; and H. Hettkamp, et al., Eur. J Biochem. 1984, 142, 85-90).

Compound **21b** was used as a starting material for the syntheses of compounds **3 - 9.** The secondary amine function of **21b** was protected with the Boc group **(22),** of which the C-1'-C 2' bond was cleaved using Pb(OAc)₄ to give the aldehyde **23.** Compound **24** obtained by reduction of the aldehyde function using DIBAL was mesylated (**25**) and treated with NaN₃ to afford **26.** During the substitution reaction, **27** was obtained as a by-product (28%), which could be converted back to **24.** The azide group was then reduced selectively in the presence of benzyl groups and the amine was acetylated to give **29.** Protecting groups were finally removed sequentially by hydrogenolysis and acid hydrolysis to give compound **3.** When the Boc group was removed first followed by hydrogenolysis, the process took a longer reaction time (1 week) and gave a mixture of **3** and **4.**

In order to obtain compounds **4** and **5,** the Boc group of **29** was deprotected **(30)** followed by *N*-methylation *via* reductive alkylation, and the benzyl groups were hydrogenolized to give **4** or **5.**

The intermediate **22** was also utilized to obtain **6** ∼ **8**. Compound **22** was converted to the 2'-acetamido compound **(37)** *via* tosylation **(33),** substitution reaction with NaN₃ **(34),** benzylation **(35),** and selective reduction of the azide function to an amine followed by acetylation **(37).** Sequential deprotection of the benzyl group and the Boc group resulted in **6.**

Compound **37** was treated with TFA to give **38** which was alkylated in the same manner as for the syntheses of **4** and **5** and finally hydrogenolized to afford **7** and **8.** The dimer **9** was obtained as the result of self-quenching under reductive amination in the presence of ammonium acetate followed by deprotection.

### Synthesis of Hexoaminidase Inhibitors

### Synthesis of Five-membered Acetamido-iminocyclitols:

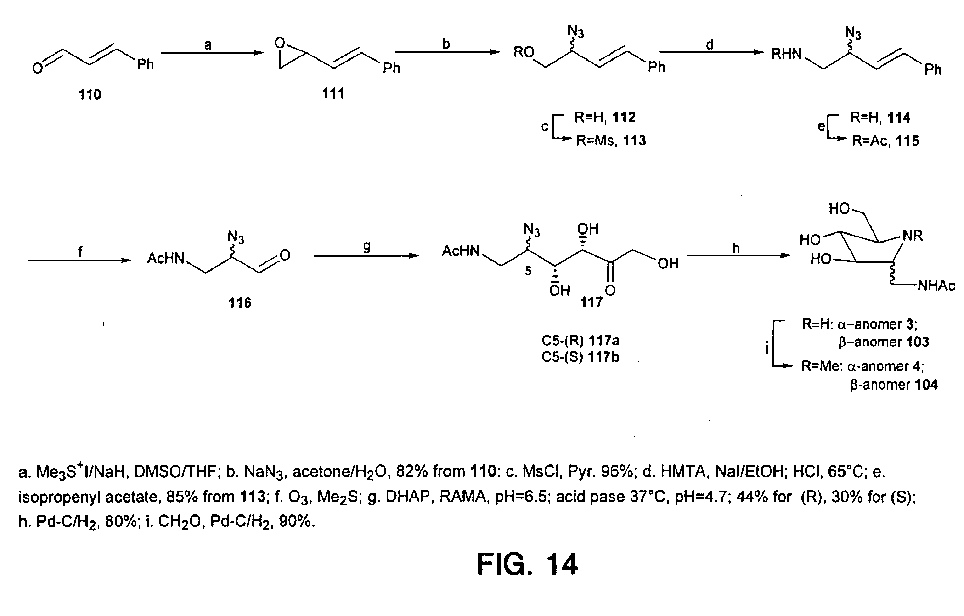

Iminocyclitols were prepared through the condensation of an aldehyde with dihydroxyacetone phosphate (DHAP) (C.-H. Wong, et al., Angew. Chem. Int. Ed. Eng. 1995, 34, 412; and H.J.M. Gijsen, et al., Chem. Rev. 1996, 96, 443). Both α and β five-membered acetamido-iminocyclitols and their derivatives were prepared following the procedures outlined in Figure 14 according to the method of Takaoka et al. (Y. Takaoka, et al., J. Org. Chem. 1993, 58, 4809).

*Trans*-cinnamaldehyde was first reacted with dimethylsulfonium methylide; which was prepared *in situ*, to get the epoxy-phenyl butene **109**. Treating with NaN₃ in acetone/water, we got the racemic hydroxyamine **110**. Mesylation of the compound **110** afforded compound **111,** which was treated with hexomethylene tetramine to yield the ammonium salt. Without purification, the crude product was converted to the primary amine **112** by the hydrolysis with concentrated HCl.

Since both α and β isomers were needed by us, no resolution step was conducted. Without purification, the amine compound **112** was acetylated with isopropenyl acetate to afford a racemic mixture **113.**

Ozonolysis of the mixture in methanol under -78°C, followed by worked up with dimethyl sulfide produced the racemic aldehydes **114.** The mixture was directly condensed with DHAP, catalyzed by FDP-aldolase. The phosphate group of the aldol products was removed by acid phospatase to yield **115**a and **115b,** which can be efficiently separated by the silica gel chromatography. Stereoselective hydrogenation afforded compound **3** and **4.**

The two five-membered iminocyclitols **3** and **4** were hydrogenated with formaldehyde in methanol/water. The *N*-methyl derivatives **103** and **104** were prepared respectively in high yield.

The *N*-methyl-β-isomer iminocyclitol **104** can also be prepared from 2,5-anhydro-2,5-imino-D-Glucitol **116** which can be produced in large quantities by the microbial oxidation of fructose (E.W. Baxter, et al., J. Org. Chem. 1994, 59, 3175). Further straightforward manipulation afforded the key intermediate **117** (C.-H. Wong, et al., J. Org. Chem. 1995, 60, 1492).

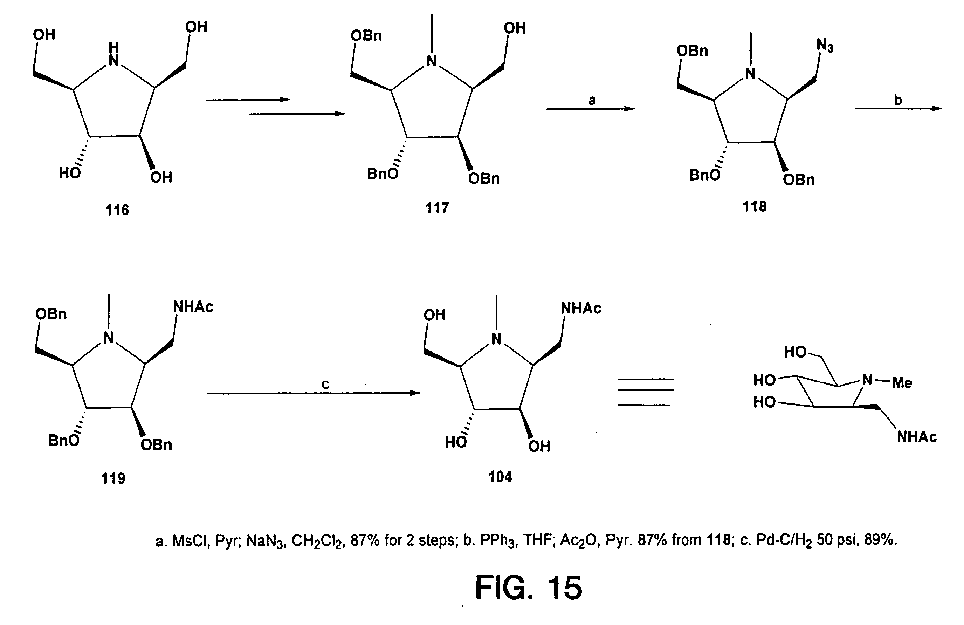

As illustrated in Figure 15, after activation the hydroxyl group in **117** with MsCl, the crude product was directly treated with NaN₃ in pyridine to afford azide compound **118.** After reduction of the azide and acetylation of the amine, the tribenzyl-protected five membered ring iminocyclitol's β-isomer **119** was yielded. The final *N*-methyl-β-acetamino-iminocyclitol **104** was prepared in high yield after complete hydrogenation.

### Synthesis of Six-membered 2-aceamido-2-deoxy-iminocyclitols:

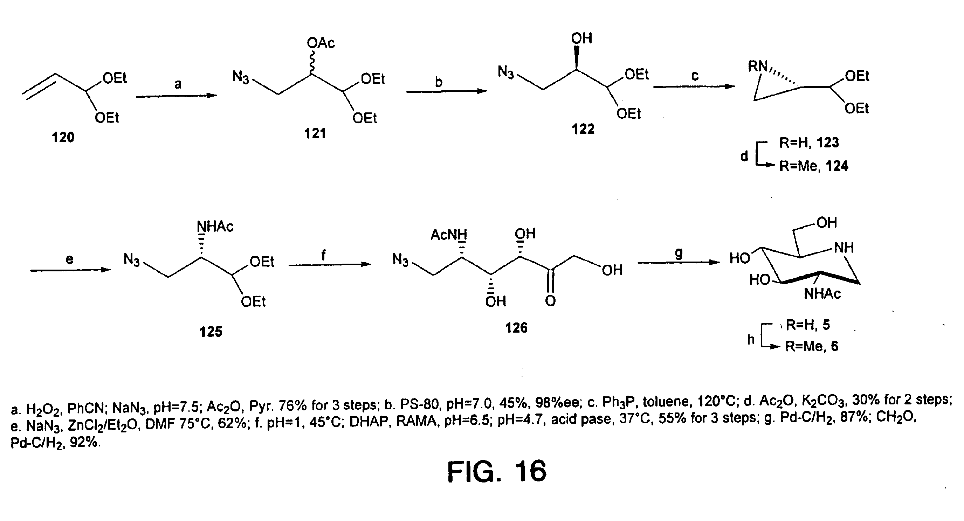

The synthesis of the six memebered ring iminocyclitols was achieved according to the method of T. Kajimoto, et al. (T. Kajimoto, et al., J. Am. Chem. Soc. 1991, 113, 6187) and as outlined in Figure 16. Starting from the commercial available compound **120,** the azide compound **121** was synthesized in a one-pot reaction. Through the resolution with the lipase PS-80, the desired enantiomer was selectively deacetylated to afford alcohol **122.** Under the classical condition, the aziridine **123** was prepared. Acetylation afforded **124.** Nucleophilic opening of the aziridine with sodium azide in the presence of ZnCl₂ gave **125** in 62% yield. After acid hydrolysis to unmask the aldehyde protecting group, the aldehyde product of compound **125** was condensed with DHAP in the presence of FDP aldolase. The phosphate group of the aldol product was then removed by acid phospatase to yield **126** as before. **126** was converted to iminocyclitol **105** via reductive amination. Following the same procedure described in the preparation of the five-membered ring iminocyclitols, *N*-methyl product **106** can also be prepared.

### Synthesis of N-Acetylhexosamine-thiazoline

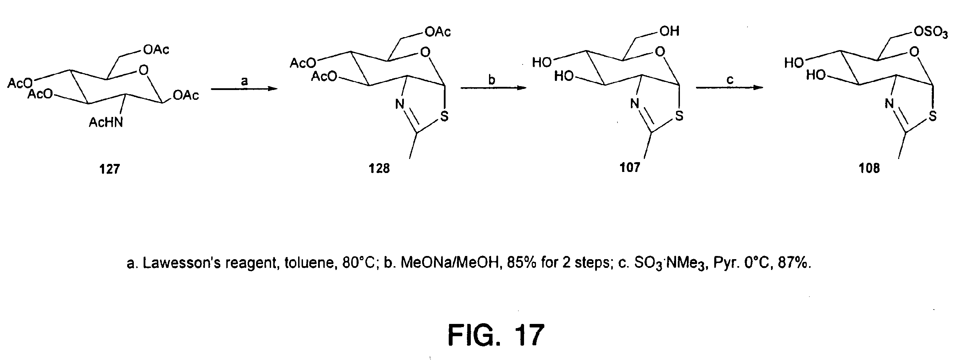

Following the procedure suggested by Knapp S. et al. (S. Knapp, et al., J. Am. Chem. Soc. 1996, 119, 6804), the target compound was synthesized according to the method outlined in as Figure 17.

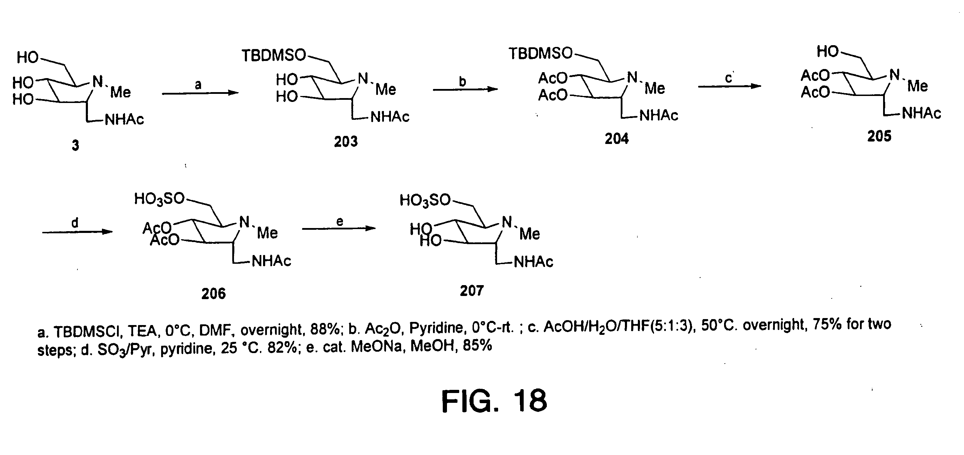

The preparation of the 6-sulfated α-iminocyclitol **207** starts from the its parent compound 3 according to the method outlined in Figure 18.

The primary hydroxyl group in 3 was first selectively protected with TBDMSCI; TBDMSOTf was found to afford a mixture of primary and secondary silyl ethers. The remaining two secondary OH groups in **203** were then protected as acetates to give the fully protected α-iminocyclitol **204.** Under acidic condition, the silyl ether was cleaved to afford 6-OH α-iminocyclitol **205** in a yield of 75% for two steps. Treating **205** with SO₃(pyridine complex in pyridine provided the diacetyl protected sulfated α-iminocyclitol **206** in 82% yield. Removal of the two acetate produced the target sulfated α-iminocyclitol **207** in a yield of 85%.

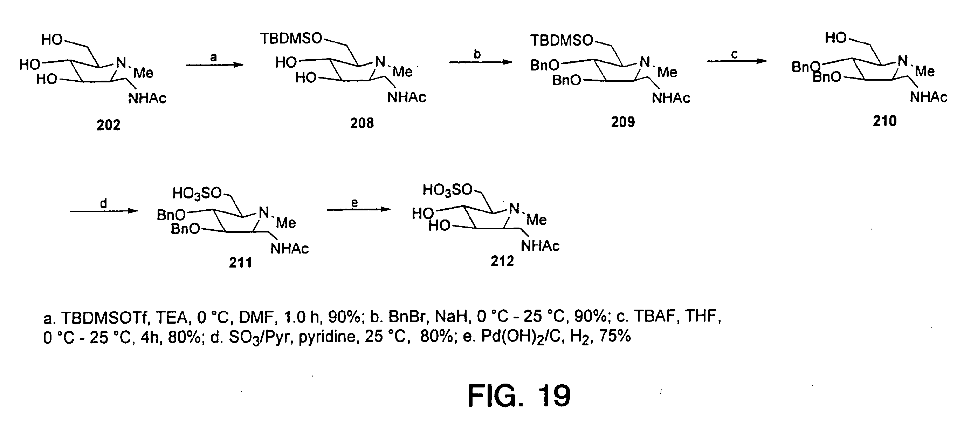

The sulfation of β-iminocyclitol **202** was provided different results **(****Fig. 19****).** The hydroxies in the β-iminocyclitol **202** seemed much less active than those in α-iminocyclitol **3.** The 6-OH doesn't react with TBDMSC1 even at elevated temperatures (130°C). However, the primary hydroxyl group of **202** reacted exclusively with TBDMSOTf at 0°C to afford 6-silyl ether protected iminocyclitol **208** in 90% yield. The different reactivities between **3** and **202** can be explained by the steric effect. There is more steric hindrance in the β-iminocyclitol **202** than in **3** due to the extending direction of N-acetyl group. The steric hindrance is enhanced by the smaller nitrogen atom relative to the oxygen which make the iminocyclitols very compact.

The silyl ether in **209** was cleaved with TBAF in THF to afford 6-OH compound **210** in 80% yield. Treating **210** with SO₃(pyridine complex in pyridine provided the benzyl protected sulfated iminocyclitol **211** in 80% yield. Hydrogenation with Pd(OH)₂/C afforded the sulfated β-iminocyclitol **212** in a yield of 75%.

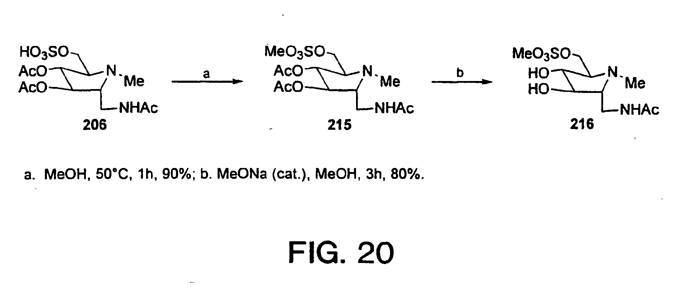

In the whole cell assay, the K*i*'s of both inhibitors **207** & **212** were in mM scale. Obviously, The negative charge of the sulfated iminocyclitols **207** and **212** made it difficult for them to reach and penetrate the cell. To deal with this problem, we employed a prodrug approach: changing the sulfated iminocyclitols to their methyl esters may allow their transportation into the cells. We hypothesized that the sulfate methyl esters can be hydrolyzed to 6-sulfate iminocyclitols at the target positions and then the 6-sulfate compounds can show their inhibition activity. Based on the prodrug approach, the sulfate methyl esters should be potent inhibitors against hexoaminidases *in vivo*. The synthesis of 6-sulfate methyl ester of α-iminocyclitols was described in Figure 20.

The 6-sulfate protected iminocyclitol **206** was methylated in methanol at 50°C in one hour. The subsequent deprotection with catalytic MeONa gave the 6-sulfate methyl ester α-iminocyclitol **216** in 80%.

The bioassay results confirmed the activity of these compounds. The Ki of the 6-sulfate methyl ester α-iminocyclitol **216** was 20nM in the whole cell assay. We also found that **216** could selectively inhibit hexoaminidase A without inhibition of hexoaminidase B. Because HexA accepted both sulfated and non-sulfated substrates while HexB only accepted non-sulfated substrates, the selective inhibitors to HexA may have less side effect on the metabolism of nonsulfated glycans while they delayed the degradation of GAG side chains, which are heavily sulfated.

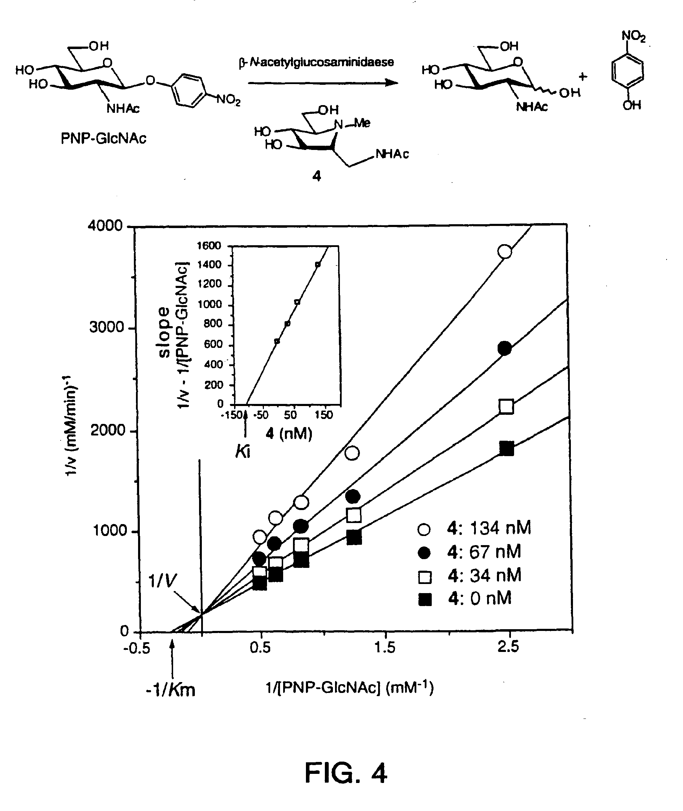

### Inhibition analysis of glycosidase inhibition activity with CZE (Figure 4)

A conventional method for the assay of glycosidase reactions is based on spectrophotometric analysis using chromogenic aglycons to detect the released chromophore directly (J. Borooah, et al., Biochem. J. 1961, 78, 106-110; D. Leaback, et al., Biochem. J. 1961, 78, 151-156; and R.G. Price, et al., Biochem. Biopys. Acta 1972, 271, 145-153) or using a substrate so the product is detected with an NADH coupled reaction ( D.K. Fitzgerald, et al., Anal. Biochem. 1970, 36, 43-61). As an alternative to radioactive analytical methods, CZE may be employed, as indicated *supra*. The CZE technique was herein employed as a general analytical method not only for glycosyltransferases but also for glycosidases. In addition, since the method relies on the peak separation, potential ambiguity arising from the possibilities of formation of byproducts can be eliminated. The analysis of transferase reactions requires peak separation of the substrate and the product before the actual kinetic analysis; however, the analysis of glycosidase reactions was more straightforward because the cleaved chromophore is usually acidic and can easily be distinguished from neutral carbohydrates. To make the CZE analysis a general method, however, a condition must be identified (usually the buffer system) that gives different migration times for the substrate and the released aglycon. This is especially necessary in the case where the released aglycon does not have a specific absorbance or fluorescence.

Initially, the total volume of assay solution was reduced because this is the only way to reduce the amount of enzyme and substrate used in the assay. The 96-well microtiter plate with a round bottom was used for the assay, and each well was sealed with tape. Thus, the assay was examined and carried out in a total volume of as little as 20 µL containing 1.76 mU of a glycosidase such as β-*N*-acetylhexosaminidase P. The electrophoresis was carried out using 50 mM borate buffer (pH 9.2 ∼ 10.2) as the electrolyte and the progress was monitored at 37°C. The injected volume of approximately 38.4 nL of a reaction mixture contained as little as 3.8 pmol of the substrate *p*-nitrophenyl (PNP) glycoside, thus the amount of PNP detected was less than 10⁻¹² mol. The peak corresponding to the *p*-nitrophenol, which appeared at around 6.5 min. in these conditions, was monitored at 405 nm.

The inhibition studies of compounds **1 - 9** against α- and β -glucosidases (from *Saccharomyces sp.* and sweet almond, respectively) were carried out. PNP-glycosides of the parent sugars were used as the substrates throughout the assay. The apparent *K*ₘ and *V*ₘₐₓ values for each substrate were calculated from the double-reciplocal plot of standard 1/ν - 1/[substrate] curve.¹⁸ The *K*ᵢ values were determined from a replot of the slopes of Lineweaver-Burk plots vs. the inhibitor concentrations. The analysis of inhibition of β-*N*-acetylglucosaminidase from bovine kidney by **4** was shown in Figure **4****,** where the apparent *K*ₘ and *V*ₘₐₓ values for PNP-GlcNAc were determined to be 4.1 mM and 6.4 µM/sec/mg, and the *K*i value of the competitive inhibitor **4** was determined to be 0.11 µM. Kinetic parameters thus obtained for the enzymes examined with other inhibitors were shown in Figure 4.

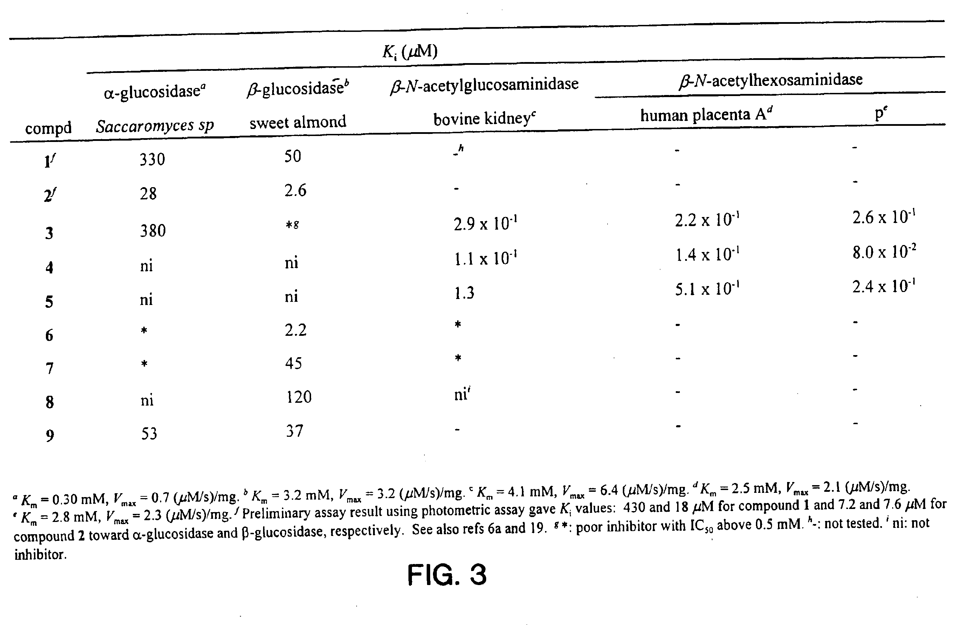

As shown in Figure 3, remarkable inhibitory specificities were observed for the compounds synthesized against glycosidases. Compounds **1** and **2** were designed and synthesized previously to inhibit β- and α-glucosidase, respectively. In our design, the side chain at position 2 in each compound is modified to mimic the aglycon part of the substrate for each enzyme. However, compound **2** showed potent inhibitory activities against both α- and β-glucosidases, whereas **1** was shown to be a weak inhibitor against both enzymes. Previously, inhibition assays of azasugars **1** and **2** using photometric assay system were carried out, which showed good agreement to the results obtained by CZE. The differences in the inhibitory activities may be explained by their conformational differences as suggested by the ¹H NMR analysis (Table 1). The N-methylated derivatives of **1** and **2** were also prepared, but exhibited weaker inhibiory activities against both enzymes (IC₅₀ > 1 mM).

Replacement of the 2'-hydroxyl group of **6** with the NHAc group had no impact on the activity towards glucosidases. It showed an identical inhibitory activity to **2,** but alkylation of the ring nitrogen had negative effects.

Compounds **3 - 5,** which lack one carbon and one hydroxyl group, showed no inhibitory activity against β-glucosidase up to 500 µM and only very weak inhibition against α-glucosidase. Instead, these compounds were found extremely potent inhibitors of *N*-acetylglucosaminidase from bovine kidney (Y. Takaoka, et al., J. Org. Chem. 1993, 58, 4809-4812) and human placenta (A and P). Also, methylation of the ring nitrogen improved the activity; however, a decrease in activity was observed with a longer *N*-alkyl substituent.

It was revealed that an acetamido group is necessary at the C-1' position of the five membered iminocyclitols in order to inhibit *N*-acetylglucosaminidase. It was also revealed that, in the case of inhibition of glucosidases, a OH group at C-1' is required, perhaps to mimic the OH-2 group of glucose. The dimeric derivative **9,** however, has an inhibitory activity at the same level as **1** and **7** toward the β-glucosidase, and also is as effective as **2** toward the α-glucosidase despite the absence of an OH group to mimic the OH-2 group of glucose. The additional moieties may have used to circumvent the lack of OH group or to give additional binding affinity (E.A. MacGregor, et al., Biochem. J. 1989, 259, 145-152).

### Inhibition assay for hexoaminidases:

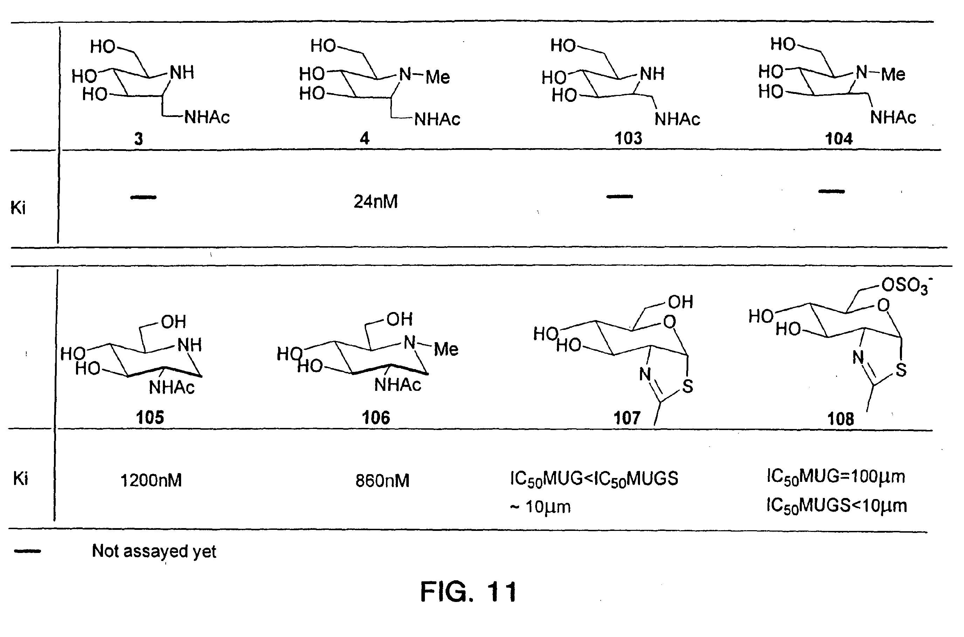

The indicated compounds synthesized above have been assayed and found to be potent inhibitors of β-*N*-acetylhexaoaminidases A from human placenta (Figure 11), especially the 5-membered iminocyclitol compound **4**(Ki=24nM). The potent inhibition can be explained by virtue of the ring nitrogen, protonated at the physiological pH, mimicking the positive charge of the oxonium ion. In addition, the transition state conformation is mimicked better by the five member ring here. The participation of 2-acetamido group is less important in this case.

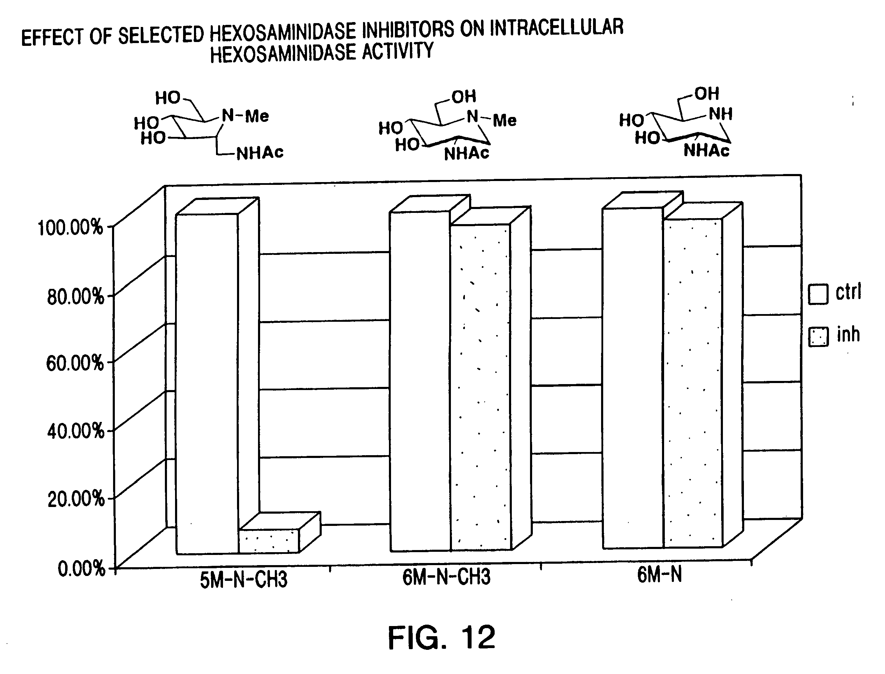

The most exciting result is shown in **Fig. 12****,** the intracellular β-*N*-acetylhexaoaminidases activity is dramatically decreased by inhibitor 4 in contrast to the six-membered iminocyclitols **105** and **106.** That suggests that the five-membered iminocyclitol 4 can penetrate through the cell membrane and inhibit the β-*N*-acetylhexoaminidases in the cytoplasm. The whole cell assay shows that compound **4** can decrease the GAGs' extracellular concentration and increase GAG's intracelllular concentration.

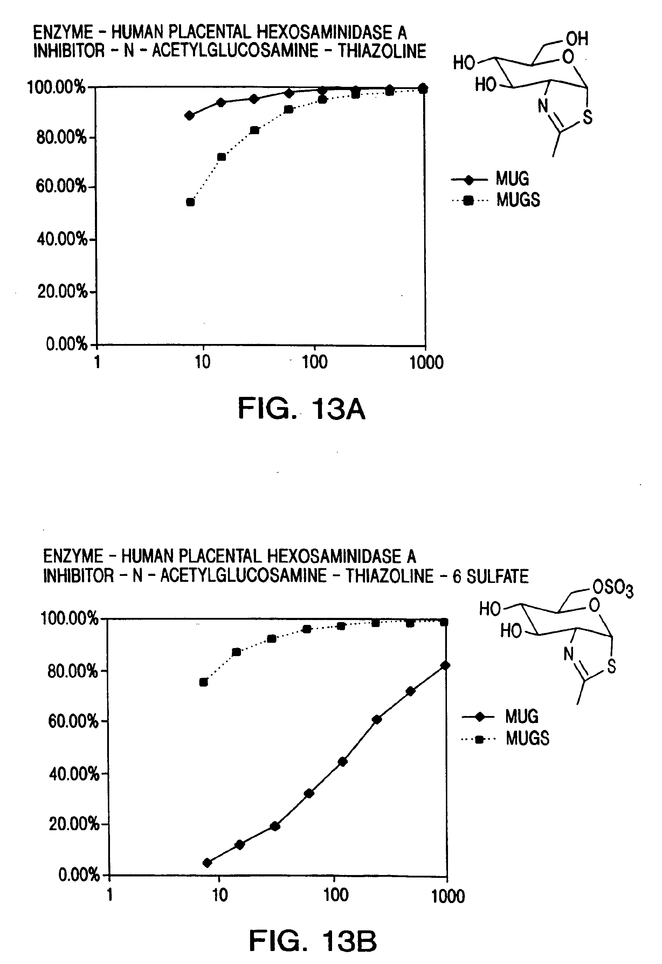

When the β-*N*-acetylhexosaminidase A was used, the sulfated thiazoline **108** was found to show higher inhibition activity towards the 6-sulfate-4-methyl-umbelliferyl-β-D-*N*-acetylglucosamine (MUGS) than to 4-methyl-umbelliferyl-β-D-*N*-acetylglucosamine (MUG). In contrast, **107** shows higher inhibition activity to MUG than to MUGS as illustrated in Figures 13A and 13B.

All the GAG chains except hyaluronic acid are sulfated (T.T. Glant, et al., J. Immunol. 1998, 28, 3812). This result implies, as a general rule, that sulfated inhibitors maybe show better inhibition activity than the nonsulfated ones for the GAG side chains.

### Experimental

### General methods for the synthesis

Dried solvents were used for all reactions. Solutions were evaporated under reduced pressure at a bath temperature not exceeding 50°C. Column chromatography was performed on silica gel or Iatro Beads (60 µ). Gel permeation chromatography was performed using Bio Gel P-2. Melting points were measured with a melting point apparatus and are uncorrected. Optical rotations were measured in a 1.0 dm tube with a polarimeter at 24±1°C. ¹H NMR (270 MHz) were recorded on solutions in CDCl₃ or D₂O using Me₄Si (δ 0.00) or DOH (δ 4.80) as the internal standard. ¹³C NMR (67.5 MHz) spectra were recorded on solutions in CDCl₃ or D₂O using Me₄Si (δ 0.00), CDCl₃ (77.00), CD₃CN (δ 118.20), or CD₃OD (δ 49.80) as the internal standard. Some key compounds were measured with a 400 MHz spectrometer as indicated. Only partial assignments were reported. The FAB mass and HR FAB mass spectra were obtained with glycerol and 3-nitrobenzylalcohol as the matrix. MALDITOF mass spectra were recorded with 2,5-dihydroxybenzoic acid as the matrix.

*Methyl (4R,5R,6R)-6-hydroxy-4.5.7-tribenzyloxy-2(E)-heptenoate* ***(11)*:** A solution of 2,3,5-tri-*O*-benzylarabinofuranose **(10)** (420 mg, 1.0 mmol) and methyl (triphenylphosphoranylidene)acetate (435 mg, 1.3 mmol) in benzene (10 mL) was heated under reflux for 12 h. After cooling, the solvent was removed *in vacuo* and the crude mixture was purified by flash column chromatography (3:1 hexane-EtOAc). The *E*-isomer **(11)** was obtained as a major product (419 mg, 88 %), [α]_{D} -4.1°(*c* 2.2, CHCl₃) along with the Z-isomer (47 mg, 10%).

*(4R,5R,6R)-4,5,7-tribenzyloxy-2(E)-hepten-1,6-diol **(12)***: To a solution of compound **11** (3.9 g, 8.19 mmol) in dry CH₂Cl₂, was added 1 M solution of DIBAL (24.6 mL, 3 eq) at 0 °C. The reaction mixture was stirred at the temperature for 1.5 h. MeOH (4 mL) was added at 0°C and the temperature was raised to r.t.. Sat. NaCl (8 mL) was added and the mixture was diluted with Et₂O (200 mL). MgSO₄ (21 g) was added and the whole mixture was stirred for 1 h, then filtered through Celite pad. The solvent was removed *in vacuo* and the crude mixture was purified by column chromatography (1:1 hexane-EtOAc) to give the alcohol **12** (3.54 g, 96.5 %), [α]_{D} -22.6°(*c* 1.0, CHCl₃).

*(4R,5R,6R)-1-tert-butyldimethylsilyloxy-4,5,7-tribenzyloxy-2(E)-hepten-6-ol **(13)***:
Compound **12** (5.17 g, 11.54 mmol) was dissolved in DMF (100 mL), to this solution was added TBDMSCl (2.09 g, 13.9 mmol), Et₃N (4 mL, 28.85 mmol) and DMAP (50 mg). The reaction mixture was stirred at r.t. for 1 h. The mixture was diluted with EtOAc and the organic layer was washed with H₂O, brine and dried with Na₂SO₄. After removal of the solvent, the residue was purified by column chromatography (10:1 hexane-EtOAc) to give **13** (6.15 g, 95 %) as a colorless oil, [α]_{D} -18.2°(*c* 1.05, CHCl₃).

*(4R,5R,6R)-1-tert-butyldimethylsilyloxy-6-[(chloromethylsulfonyl)-oxy]-4,5,7-tribenzyloxy-2(E)-heptene* ***(14)*:** A solution of compound 13 (480 mg, 0.85 mmol) and chloromethylsulfonyl chloride (91 mL, 1.0 mmol) in pyridine (2 mL) was stirred at r.t. for 0.5 h, then the mixture was diluted with EtOAc and washed with H₂O and brine, and dried over Mg₂SO₄. After removal of the solvent, the residue was purified by column chromatography (10:1 hexane-EtOAc) to give **14** (564 mg, 98 %) as a colorless oil.

*(4R,5R,6S)-6-acetoxy-1-tert-butyldimethylsilyloxy-4,5,7-tribenzyl-oxy-2(E)-heptene **(15)***:
The stirred mixture of compound **14** (2.40 g, 3.56 mmol), CsOAc (3.40 g, 5 eq), and 18-crown-6 (950 mg, 1 eq) was heated under reflux in toluene (80 mL) for 12 h. After cooling to r.t., the reaction mixture was washed with H₂O and brine, dried over Na₂SO₄, and the solvent was removed *in vacuo*. The crude material was purified by column chromatography (10:1 hexane-EtOAc) to afford **15** (1.98 g, 92 %) as a colorless oil.
*(4R,5R,6S)-1-tert-butyldimethylsilyloxy-4,5,7-tribenzyloxy-2(E)-hepten-6-ol **(16)***:

Compound **15** (120 mg, 0.2 mmol) was dissolved with MeOH (2 mL) and treated with 1 M solution of NaOMe (600 mL, 3 eq) at r.t. for 1 h. The solvent was removed and the residue was diluted with EtOAc, washed with H₂O and brine, and dried over Na₂SO₄. Purification by column chromatography using 5:1 hexane-EtOAc as an eluent gave **16** (107 mg, 96 %), [α]_{D} -5.4° (*c* 1.13, CHCl₃).

*(4R,5R,6S)-1-tert-butyldimethylsilyloxy-6-[(chloro-methylsulfonyl)-oxo]-4,5,7-tribenzyloxy-2(E)-heptene **(17)***: A mixture of compound **16** (1.82 g, 3.23 mmol) and chloromethylsulfonyl chloride (342 mL, 3.83 mmol) in pyridine (14 mL) was stirred at r.t. for 0.5 h. The mixture was diluted with EtOAc, washed with H₂O and brine, and dried over Na₂SO₄. After removal of the solvent, the residue was purified by column chromatography (15:1 hexane-EtOAc) to give **17** (1.99 g, 92 %) as a colorless oil.

*(4R,SR,6S)-6-[(chloromethylsulfonyl)oxy]-4,5,7-tribenzyloxy-2(E)-hepten-1-ol **(18)***: A solution of compound **17** (1.0 g, 1.48 mmol) in THF (5 mL) was treated with 1N HCl (5 mL) at r.t. for 8 h. The THF was removed and the mixture was diluted with EtOAc and the organic layer was washed with aq. Na₂CO₃, H₂O and brine. After concentration, the residue was purified by column chromatography (3:1 hexane-EtOAc) to aford **18** (790 mg, 95 %) as a colorless oil.

*(2S,3R,4S,5R,6S)-6-[(chloromethylsulfonyl)oxy]-2,3-epoxy-4,5,7-tribenzyloxy-2(E)-hepten-1-ol **(19a)***: A solution of Ti(O-i-Pr)₄ (365 µL, 1.26 mmol) and L-(+)-diethyltartrate (210 µL, 1.26 mmol) in CH₂Cl₂ (4 mL) was stirred at -25°C for 0.5 h in the presence of MS 4A (activated at 150C by microwave oven). To this mixture was added a solution of compound **18** (350 mg, 0.63 mmol) in CH₂Cl₂ (1 mL), and the mixture was stirred at the temperature for 0.5 h.
A solution of *t*-BuOOH (5 *M*, 365 µL, 1.89 mmol) was added and the mixture was stirred at the same temperature for 48 h. A solution of 10% tartaric acid was added at -25°C and stirred for 0.5 h at the temperature, and for 0.5 h at r.t.. The solution was filtered through a Celite pad and the filtrate was washed with H₂O and brine, and the solvent was removed. The residue was dissolved in Et₂O (20 mL) and stirred with 10 % NaOH solution at 0°C for 0.5 h. The organic layer was washed with H₂O and brine, dried and concentgrated. The crude mixture was purified by column chromatography (1:1 hexane-EtOAc) to afford **19a** (270 mg, 75 %) as a colorless oil.
*(2S,3R,4S,5R,6R)-6-azidle-2,3-epoxy-4,5,7-tribenzyloxy-2(E)-hepten-1-ol **(20a)***:
Compound **19a** (225 mg, 0.39 mmol) was dissolved with DMF (3 mL) and treated with NaN₃ (51 mg, 0.78 mmol) at 70°C for 0.5 h. After cooling to r.t., the mixture was diluted with EtOAc and washed with H₂O and brine, dried and the solvent was removed. The residue was purified by column chromatography (2:1 hexane-EtOAc) to give **20a** (157 mg, 82 %) as a colorless oil;
IR 2200 cm⁻¹.

*(1'R,2S,3R,4R,5R)-3,4-dibenzyloxy-5-benzyloxymethyl-2-[1',2'-dihydroxy-ethyl]-pyrrolidine **(21a)***: A solution of compound **20a** (175 mg, 0.36 mmol) and triphenylphosphine (113 mg, 0.43 mmol) in THF (5 mL) containing ca. 0.5 % H₂O was stirred at r.t. for 48 h. After removal of the solvent, the residual mixture was purified by column chromatography (20:1 CHCl₃-MeOH) to give **21a** (137 mg, 82 %).

*(2R,3S,4R,5R,1'R)-5-hydroxymethyl-3,4-dihydroxy-2-(1',2'-dihydroxy)ethyl-pyrrolidine **(1)***: A solution of **21a** (58 mg, 0.13 mmol) in MeOH (1 mL) was stirred with Pd/C under H₂ atmosphere at r.t. for 48 h. The crude material, obtained after removal of the catalyt and solvent, was purified by column chromatography (6:4:1 CHCl₃-MeOH-H₂O) to afford **1** (17 mg, 70 %); [α]_{D} +11°(*c* 0.1, D₂O).

*(2R,3S,4S,SR,6S)-6-[(chloromethylsulfonyl)oxy]-2,3-epoxy-4,5,7-tribenzyloxy-2(E)-hepten-1-ol **(19b)***: Compound **19b** was synthesized using **18** (250 mg, 0.43 mmol), a solution of Ti(O-i-Pr)₄ (260 µL, 0.86 mmol), D-(-)-diethyltartrate (150 µL, 0.86 mmol), a solution of *t*-BuOOH ( 5 *M*, 260 µL, 1.29 mmol), a solution of 10 % tartaric acid, MS 4A and CH₂Cl₂ (4 mL, total volume) as described for the synthesis of compound **19a;** Yield: **19b** (235 mg, 91 %), a colorless oil.
*(2R,3S,4S.5R,6R)-6-azide-2.3-epoxy-4,5,7-triberazyloxy-2(E)-hepten-1-ol **(20b)***:
Compound **20b** was synthesized using **19b** (195 mg, 0.34 mmol), NaN₃ (44 mg, 0.68 mmol) and DMF (3 mL) as described for the synthesis of **20a;** Yield: **20b** (144 mg, 87 %), a colorless oil: [α]_{D} -29.3°(*c* 1, CHCl₃); IR 2170 cm⁻¹.

*(2R,3S.4R,5R,1'R)-5-benzyloxymethyl-3,4-dibenzyloxy-2-(1',2'-dihydroy)ethylpyrrolidine **(21b)***: Compound **21b** was synthesized using **20b** (3.44 g, 7.03 mmol), triphenylphosphine (2.21 g, 8.44 mmol) and THF (36 mL) as described for the synthesis of **21a;**
Yield: **21b** (2.98 g, 91 %).

*(1R,2S,3R,4R,5R)-3,4-dihydroxy-2-[1',2'-dihydroxy-ethyl]-5-hydroxymethyl-pyrrolidine* ***(2)*:** Compound **2** was obtained by hydrogenolysis of **21b** (100 mg, 0.22 mmol) was carried out as described for the synthesis of **1** in MeOH (1 mL) to afford **2** (30 mg, 72 %); [α]_{D} +25.6°(*c* 0.3, D₂O).

*(1"R,2R,3R,4R,5R)-N-Butyloxycarbonyl-[3,4-dibenzyloxy-5-benzyloxymethyl-2-(1',2'-dihydioxy-ethyl)]-pyrrolidine **(22)***: To a solution of **21** (336 mg, 0.73 mmol) in CH₂Cl₂ (7 mL) and Et₃N (121 µL, 0.87 mmol), (Boc)₂O (412 µL, 1.74 mmol) was added at 0°C and the mixture was stirred at r.t. for 20 h. The reaction mixture was diluted with CH₂Cl₂ and washed with 10% citric acid, sat. NaHCO₃, and water, dried over MgSO₄, and concentrated. The resulting material was purified on a column of silica gel eluted with hexane-EtOAc (3:1) to afford **22** (374 mg, 91 %), [α]_{D} = -31.3°(*c* 1.0, CHCl₃).

*(2S,3R,4R,5R)-N-Butyloxycarbonyl-(3,4-dibenzyloxy-5-benzyloxymethyl)-pyrrolidime-2-carbaldehyde **(23)***: To a solution of compound **22** (740 mg, 1.3 mmol) in toluene (13 mL), Pb(OAc)₄ (959 mg, 2.0 mmol) was added. The reaction mixture was stirred for 1.5 h at r.t., then diluted with Et₂O, filtered through a Celite pad, and concentrated. The resulting residue was purified on a column of silica gel eluted with 8:1 hexane-EtOAc to afford **23** (684.8 mg, ∼quant.); [α]_{D} -56.6°(*c* 1.0, CHCl₃). NMR (CDCl₃) analysis showed that **23** existed as two conformational isomers which were designated to be "major" and "minor". Major / Minor = 10/7.

*(2R, 3R, 4R, 5R)-N-Butyloxycarbonyl-(3, 4-dibenzyloxy-5-benzyloxymethyl-2-hydroxymethyl)-pyrrolidine **(24)***: To a solution **of 23** (1.17 g, 2.2 mmol) in CH₂Cl₂ (15 ml) cooled to 0°C was added 0.98 M di-*iso*-butylaluminum hydride (DIBAL) in hexane (2.7 mL, 2.6 mmol) and the resulting mixture was stirred for 0.5 h until completion. MeOH (1 mL) was added to the mixture and was stirred at r.t. for 0.5 h. The mixture was diluted with Et₂O₂ washed with brine, dried over MgSO₄, and concentrated to give a syrup, which was purified by flush column chromatography using 4:1 hexane-EtOAc as the eluent to give **24** (1.14 g, 97%); [α]_{D} -42°(*c* 1.0, CHCl₃). Major / Minor = 20/7.

*(2R,3R,4R,5R)-N-Butyloxycarbonyl-(2-azidomethyl-3,4-dibenzyloxy-5-henzyloxymethyl)-pyrrolidine **(26)*** and *(2R,3R,4R,5R)-(3,4-dibenzyloxy-5-benzyloxymethyl)-pyrrolidino-[1,2,c]-oxazole- 3-one **(27)***: To a solution of compound **24** (371 mg, 0.70 mmol) in CH₂Cl₂ (7 mL) was added MsCl (81 µL, 1.04 mmol) and Et₃N (145 µL, 1.04 mmol) at 0°C. The mixture was stirred at r.t. for 2 h, diluted with EtOAc, washed successively with *N* HCl, sat.NaHCO₃, water, and brine, then dried over MgSO₄ and concentrated. The resulting syrup was purified on a column of silica gel eluted with 5:1 hexane-EtOAc to give mesyl ester **25** [Rf 0.48 (2:1 hexane-EtOAc); 424 mg, quant.] which was then dissolved in DMF (9 mL). To this solution was added NaN₃ (451 mg, 6.9 mmol), and the mixture was stirred at 70°C for 35 h. The mixture was concentrated to about half volume and diluted with EtOAc, washed with water, dried over MgSO₄, and concentrated. The residue was purified on a column of silica gel using 9:1 and 3:1 hexane-EtOAc as eluent. The former eluent afforded the desired **26** [Rf 0.76 (2:1 hexane-EtOAc); 253 mg, 65%] (Major / Minor = 5/4) and the latter gave oxazolone **27** [Rf 0.32 (2:1 hexane-EtOAc); 94 mg, 28%]. Physical data for compound **26**: [α]_{D} -47.5°(*c* 1.0, CHCl₃); Complex signals were obtained due to the existance of two conformational isomers at almost 1:1 ratio, and only chemical shifts were reported. Physical data for compound **27:** [α]_{D} = +4°(*c* 1.0, CHCl₃).

*(2R,3R,4R,5R)-N-Butyloxycarbonyl-(2-aminomethyl-3,4-dihenzyloxy-5-benzyloxymethyl)-pyrrolidine **(28)*** : A mixture of compound **26** (65 mg, 0.12 mmol) and 5% Pd on C (ca. 20 mg) in MeOH (2.5 mL) was stirred under H₂ atmosphere at r.t. for 1.5 h until completion [Rf 0.49 (9:1 CHCl₃-MeOH)]. The reaction mixture was filtered to remove the catalyst, then concentrated to dryness to afford **28** (59 mg, Major / Minor = 4/3); [α]_{D} -48.1 °(*c* 1.6, CHCl₃).

*(2R,3R,4R,5R)-N-Butyloxycarbonyol-(2-acetamidomethyl-3,4-dibenzyloxy-5-benzyloxymethyl)-pyrrolidine **(29)***: The amine **28** was acetylated using Ac₂O (30 µL) and Pyr. (2 mL) to yield **29** (55.7 mg, 83%, Major / Minor = 10/7) after purification on a column of silica gel using a 20:1 mixture of CHCl₃-MeOH as eluent; [α]_{D} -4.6°(*c* 0.5, CHCl₃).

*(2R,3R,4R,5R)-2-Acetamidomethyl-3,4-dihydroxy-5-hydroxymethyl-pyrrolidine **(3)***: A solution containing compound **29** (11.7 mg, 0.038 mmol) in MeOH (1.5 mL) was acidified with 0.1N HCl to pH 4-5, and was added a catalytic amount of 5% Pd on C. The reaction mixture was stirred under H₂ atmosphere at r.t. for over night. Filtration and evaporation of the solvent afforded a syrup quantitatively, which was then treated with TFA-H₂O (9:1 v/v, 300 µL) and the solution was kept at r.t. for 1 h. The mixture was neutralized to pH 8 using 28% NH₃ and concentrated. The resulting residue was purified on a column of latro Beads using a 9:2:1 mixture of *i*-PrOH-28% NH₃-H₂O to afford 15 mg of salt form which was treated with Dowex 1X8 (OH⁻) to give **3** (7 mg, 81%). **3** was further purified for the inhibition assay using Sep-Pak PLUS CM, regenerated with M HCl (10 mL) and water (20 mL), eluted with water (20 mL) and 10% NH₃-H₂O (10 mL). The latter eluent containing **3** was filtered through a Millex GV filter and lyophilized.

*(2R,3R,4R,5R)-2-Acetamidomethyl-3,4-dibenzyloxy-5-benzyloxymethyl-pyrrolidine **(30)***: Compound **29** (142 mg, 0.25 mmol) was treated with TFA-H₂O (95:5 v/v, 1.2 mL) at r.t. for 2 h. The resulting solution was neutralized with sat. NaHCO₃ to pH 7 and extracted with EtOAc. The combined organic layers were dried over MgSO₄, filtered, evaporated, and purified on a column of silica gel eluted with CHCl₃-MeOH (20:1) to afford **30** (113 mg, 96%); [α]_{D} +32.3°(*c* 1.3, CHCl₃).

*(2R,3R,4R,5R)-N-Methyl-(2-acetamidomethyl-3,4-dibenyloxy-5-benzyloxymethyl)-pyrrolidine **(31)***: To a solution of **30** (16 mg, 0.034 mmol) in MeOH (0.5 mL) at 0°C was added 37% folmaldehyde solution (5.1 mL, 0.068 mmol) and NaBH₃CN (4.2 mg, 0.068 mmol). The mixture was stirred at r.t. for overnight. The reaction mixture was added H₂O, extracted with CHCl₃ and dried with MgSO₄. After removal of the solvent, the residue was purified by preparative TLC (CHCl₃-MeOH 9:1) to yield **31** (12 mg, 71%); [α]_{D} -10.8°(*c* 0.3, CHCl₃).

*(2R,3R,4R,5R)-N-Buhyl-(2-acetamidomethyl-3,4-dibenzyloxy-5-henzyloxymethyl)-pyrrolidine **(32)***: Compound **32** was synthesized according to the procedure described for the synthesis of **31** using *n*-butanal instead of formaldehyde; Yield: 74%, [α]_{D} -35° (*c* 0.56, CHCl₃).

*(2R,3R,4R,5R)-N-Methyl-(2-acetamidomethyl-3,4-dihydroxy-5-hydroxymethyl)-pyrrolidine **(4)***: Compound **31** (18.7 mg, 0.038 mmol) was dissolved in MeOH (1.5 mL), and to this solution was acidified with 0.1N HCl to pH 4-5 and added a catalytic amount of 5% Pd on C. The reaction mixture was stirred under H₂ atomosphere at r.t. for overnight. The catalyst was removed by filtration, and the solvent was concentrated under vaccum. The residue was purified on a column of latro Beads using a 9:2:1 mixture of *i*-PrOH-28% NH₃-H₂O and treated with Dowex 1X8 (OH⁻) to give **4** (6.6 mg, 80%); [α]_{D}-26.6°(*c* 0.5, MeOH).

*(2R,3R,4R,5R)-N-Buthyl-(2-acetamidomethyl-3,4-dihydroxy-5-hydroxymethyl)-pyrrolidine **(5)***: Compound **5** was synthesized in 85% from **30** according to the procedure described for the synthesis of compound **4.**

*(1'R,2R,3R,4R,5R)-N-Butyloxycarbonyl-[3,4-dibenzyloxy-5-benzyloxymethyl-2-(2'-azido-1'-hydroxy-ethyl)]-pyrrolidine **(34)***: To a solution of compound **22** (383 mg, 0.68 mmol) dissolved in Pyr. (7 mL) was added TsCl (194 mg, 1.02 mmol). The reaction mixture was stirred at r.t. for 37 h. H₂O was added to the mixture and stirred for 5 min. The mixture was diluted with EtOAc, washed with *N* HCl, water, sat.NaHCO₃, and brine, dried over MgSO₄, and concentrated. The residue was purified on a column of silica gel (4:1 hexane-EtOAc) to give **33** [Rf 0.49 (2:1 hexane-EtOAc); 417 mg, 85%], which was then dissolved in DMF (4 mL) and the solution was added NaN₃ (151 mg, 2.33 mmol). The mixture was stirred at 70°C for 4 h, H₂O was added. After stirring for 5 min, the mixture was diluted with EtOAc, washed with brine, dried over MgSO₄, and concentrated. The residue was purified on a column of silica gel using 8:1 hexane-EtOAc as an eluent to afford **34** (235 mg, 69%, Major / Minor = 5/2); [α]_{D} -23.4°(*c* 1.0, CHCl₃).

*(1'R, 2R, 3R,4R, 5R)-N-Butyloxycarbonyl-[2-(2'-azido-1'-benzyloxy-ethyl)-3,4-dibenzyloxxy-5-benzyloxymethyl]-pyrrolidine **(35)***: To a solution of **34** (132 mg, 0.22 mmol) in DMF (3 mL) was successively added Ag₂O (208 mg, 0.9 mmol), BnBr (107 µL, 0.9 mmol), and KI (74 mg, 0.45 mmol) at 0°C. The reaction mixture was stirred at r.t. for 7 h, then added Et₂O-water and stirred for 10 min. After filtration through a Celite pad, the mixture was extracted with Et₂O, the organic layer was separated, dried over MgSO₄, and concentrated. The residue was purified on a column of silica gel using 10:1 hexane-EtOAc as an eluent to yield **35** (139 mg, 93%); [α]_{D} -30.7°(*c* 1.06, CHCl₃)

*(1'R,2R,3R,4R,5R)-N-Butvloxycarbonyl-[2-(2'-amino-1'-benzyloxy-ethyl)-3,4-dibenzyloxy-5-benzyloxymethyl]-pyrrolidine **(36)***: To a solution of compound **35** (29 mg, 0.043 mmol) in MeOH (2 mL) was added 5% Pd on C (ca. 20 mg). The mixture was stirred under H₂ atomosphere at r.t. for 1 h. Filtration and concentration afforded **36** (25 mg); [α]_{D} -30.3°(*c* 0.8, CHCl₃).

*(1'R,2R,3R,4R,5R)-N-Butyloxycarbonyl-[2-(2'-acetamido-1'-benzyloxy-ethyl)-3,4-dibenzyloxy-5-benzyloxymethyl]-pyrrolidine **(37)***: The amine **36** was redissolved in pyridine (1.5 mL), and Ac₂O (0.5 mL) was added to the solution. The mixture was kept at r.t. for overnight and concentrated to dryness. The residue was purified on preparative TLC using a 10:1 mixture of CHCl₃-MeOH as mobile phase to afford **37** (25 mg, 84%); [α]_{D} +2.6°(*c* 0.4. CHCl₃).

*(1'R,2R,3R,4R,5R)-2-(2'-acetamido-1'-hydroxy-ethyl)-3,4-dihydroxy-5-hydroxymethylpyrrolidine **(6)***: To a solution of compound **37** (41 mg, 0.059 mmol) in MeOH (1.5 mL), 0.1 N HCl (0.2 mL) and 5% Pd on C (25 mg) were added and the mixture was stirred under H₂ atomosphere at r.t. for overnight. Filtration and evaporation of the solvent afforded a syrup quantitatively, which was then treated with TFA-H₂O (9:1) at r.t. for 2 h. The mixture was adjusted to pH 8 using NH₃ (28%) and concentrated to give a syrup, which was purified on a column of Iatoro Beads using a 9:1:2 mixture of *i*-PrOH-NH₃(28%)-H₂O to yield a TFA salt (14.5 mg). The residue was treated with Dowex 1X8 (OH⁻) and eluted with water to give **6** (13.7 mg, quant.); [α]_{D} +30.6°(*c* 0.69, MeOH).

*(1'R,2R,3R,4R,5R)-2-(2'-acetamido-1'-benzyloxy-ethyl)-3,4-dibenzyloxy-5-benzyloxymethyl-pyrrolidine **(38)***: Compound **38** was synthesized from **37** according to the procedure described for the synthesis of compound **30.**

*(1'R,2R,3R,4R,5R)-N-Methyl-[2-(2'-acetamido-1'-hydroxy-ethyl)-3,4-dihydroxy-5-hydroxymethyl]-pyrrolidine **(7)***: Compound **7** was synthesized in 86% from **38** according to the procedure described for the synthesis of compound **4.**

*(1'R,2R,3R,4R,5R)-N-Ethyl-[2-(2'-acetamido-1'-hydroxy-ethyl)-3,4-dihydroxy-5-hydroxymethyl]-pyrrolidine **(8)***: Compound **8** was synthesized in 83% from **38** according to the procedure described for the synthesis of compound **4.**

*N, N-Di-{[(2R,3R,4R,5R)-(3,4-dibenzyloxy-5-benzyloxymethyl)pyrrolidine]methyl} amine **(39)**:* Ammmonium acetate (59 mg, 0.77 mmol) was added to a solution of aldehyde **23** (41 mg, 0.077 mmol) in MeOH (1 mL), then sodium cyanoborohydride (5.3 mg, 0.085 mmol) was added to the solution at r.t.. The mixture was stirred for 22 h, then concentrated and extracted with CHCl₃. The organic layer was washed with sat. NaHCO₃ and H₂O, and dried over MgSO₄. The solvent was evaporatied *in vacuo* and the residue was purified by preparative TLC (2:1 hexane-EtOAc) to give **39** [*R*f 0.46 (2:1 hexane-EtOAc); 30.4 mg, 75%]; [α]_{D} -58°(*c* 0.75, CHCl₃).

*N-Butyloxycarbonyl-N,N- di-{[(2R,3R,4R,5R)-(3,4-dibenzyloxy-5-benzyloxymethyl)-pyrrolidine]methyl} amine **(40)*** and *N,N- di-{[(2R,3R,4R,5R)-(3,4-hydroxy-5-hydroxymethyl)-pyrrolidine]methyl} amine **(9)***: To a solution of **39** (32 mg, 0.03 mmol) in CH₂Cl₂ (2 mL) and Et₃N (5 µL, 0.036 mmol), (Boc)₂O (17 µL, 0.073 mmol) was added at 0°C and the mixture was stirred at r.t. for overnight. The reaction mixture was diluted with CH₂Cl₂ and was washed with 10% citric acid, sat.NaHCO₃, and water, dried over MgSO₄, and concentrated. The resulting material was purified by preparative TLC (2:1 hexane-EtOA ) to afford **40** [*R*f 0.69 (2:1 hexane-EtOAc), 33 mg, 97%]. To the solution of compound **40** (18mg, 0.016 mmol) dissolved in MeOH (1.5 mL), acidified to pH 4-5 with 0.1N HCl, was added 5% Pd on C (ca.10 mg). The mixture was srirred under H₂ atomosphere at r.t. for over night. The catalyst was filtered off and the filtrate was concentrated. The residue was treated with TFA-H₂O (9:1, 300 µL) at r.t. for 3 h. The mixture was adjusted to pH 8 using NH₃ (28%) and concentrated to leave a syrup, which was purified on a column of latro Beads using 9:1:2 i-PrOH-NH₃(28%)-H₂O. The obtained material was finally treated with Dowex 1X8 (OH⁻) eluted with water to give **9** after concentration (4.6 mg, 94%); [α]_{D} +55°(*c* 0.42, MeOH).

*3-(E)-2-Azido-4-phenyl-1-butenol* **(110)**: NaH (11g, 95% mineral oil dispersion) was washed with hexane and dried over vacuum. Under argon, dry THF (220ml) and dry DMSO (220ml) were added and the reaction mixture was cooled in ice/salt bath. A solution of trimethylsulfonium iodide (84.86g) in 300 ml DMSO was added over 10 min. After the addition was complete. *Trans-* cinnamaldehyde **108** (18.5g) was added in one portion. The reaction mixture was stirred at 0°C for 40min and at room temperature for additional 2h. The reaction mixture was slowly quenched with 800ml water and ice and extract with methylene chloride (4(500ml). The combined organic layer were washed with water (2(500ml), dried over K₂CO₃., filtered, and concentrated to afford a brown liquid. Without purification, the liquid was dissolved in 220ml acetone and 110ml water. 20g NaN₃ was added., and then the mixture was refluxed gently for 3h. The reaction mixture was acidified by the addition of ammonium chloride (5.0g) and stirred for an additional 10min at room temperature. Water(50ml) was added, and the acetone was removed in vacuum,. The aqueous residue was extracted with methylene chloride, dried over Na₂SO₄, filtered, and concentrated. Purified by silica gel chromatography (hexane/ ethyl acetate=5:1) to yield 24.2g yellow liquid **110** (82% from **109**).

*Mesylation of compound* **110**: To a solution of 20g **110** in 100ml pyridine was added MsCl 9.8ml, and the mixture was stirred at room temperature for 1.5h, The reaction mixture was extracted with methylene chloride. After routine work up, flash chromatography (hexanes/EtOAc=4:1) afforded 31.0g(96%) of **111.**

*(E)-1-amine-2-aizde-4-phenyl-3-butene* **112:** An ethanol solution (200ml) of **111** (16.5g) was slowly added to a suspension of 9.52g hexamethylenetetraamine and 10.2g Nal. The reaction mixture was stirred for 4 days. The precipitate (26g) that appeared were collected by filtration and was used directly to the next reaction. To the precipitates was added 270ml ethanol, 27ml 12N hydrochloric acid. The mixture was heated at 65°C for 20min. After the reaction was complete, the white precipitate was removed by filtration. The filtrate was adjusted to pH 8.0 with 10% NaOH and extracted with EtOAc. The organic layer was washed with water and dried over MgSO₄ and concentrated. A small portion of the residue was purified by silica gel chromatography (CHCl₃/MeOH=20:1).

*(E)-N-acetyl-2-azide-4-phenyl-3-butene* **113**: 300ml isopropenyl acetate was added to the solution of 12.4g **112** in 200ml EtOAc, and the mixture was stirred for overnight at room temperature. After the reaction was complete, the organic solvent was removed under vacuum and the residue was purified by silica gel chromatography (Hexanes/EtOAc=1:1) to yield **113** 11.7g (85% from **111**).

*Compound* **115a** *and* **115b**: Under -78°C, a mixture containing 1.50g **113** in 200ml methanol was injected ozone gas until the color of the solution of the reaction mixture became pale blue. After bubbling Ar to remove excess amounts of ozone, the ozonide was treated with DMS (35ml) for 1 day. Methanol was evaporated and the residue was used directly in the next step without purification. To the aqueous solution of the aldehyde was added 5ml DHAP (424mM), and the pH was adjusted to 6.5 with 1N HCl. Rabbit muscle FDP aldolase(1000units) was added to this solution and the mixture was stirred slowly at room temperature until DHAP was consumed completely based on the enzymatic assay²⁰. The mixture was passed through AG 1(8 resin (HCO²⁻) (2.4(50cm) and eluted with water(1L) 0.1M NaCl(1L), and 0.4M NaCl(1L) solution, successively. The pH value of the fraction of 0.4M NaCl solution was adjusted to 4.7 with 1N HCl, 1000 units acid phosphatase (from sweet potato, type X) was added, and the mixture was stirred slowly at 37°C for I day. The pH was adjusted to 7.0 and the water was removed under reduced pressure. The residue was treated with MeOH, and the soluble portion was collected and purified by silica gel chromatography (CHCl₃/MeOH/H₂O= 10:1:0.1) to yield 230mg **115a** (44% yield based on DHAP) and 156mg **115b** (30% yield based on DHAP)

*Compound* **3** *or* **103**: A solution of compound **115a** 24mg or **115b** 14mg in 3ml ethanol was hydrogenated with 10% Pd/C (5mg) under 50psi of hydrogen for 1 day. The catalyst was removed by filtration and the solvent was evaporated under vacuum. The residue was purified by silica gel chromatography (CHCl₃/MeOH/H₂O=6:4:0.7)) to yield 16mg **3**(80%) or 9mg **103**(80%).

*N-methyl five-membered ring iminocyclitols* **4, 104**: A solution containing 30mg of **3 or 103,** formaldehyde(300ul, 37%wt solution), and 10mg of 10% Pd-C was hydrogenated under 50psi of hydrogen in 5ml of methanol/water(1:1 solution) for 24h. the solvent was removed under reduced pressure, Silica gel chromatography(CHCl₃/MeOH/H₂O=6:4:0.7) afforded **4** or **104** 28mg (90%) respectively.

*1-Azido-5(R)-(Benzyloxymethyl)-3(R),4(R)-bis(benzyloxy)-2(S)-(hydroxymethyl)-N-methyl-pyrrollidine* **(118):** 170mg of **117** and 122ul pyridine was dissolved in 5ml methylene chloride, under 0°C degree, 11ul MsCl was added. The reaction mixture was stirred for 2h at room temperature. The solvent was removed under high vacuum. Without purification, The residue was dissolved in 20ml DMF. 200mg NaN₃ and 60.5mg Nal were added to this solution. The reaction mixture was stirred at room temperature for 5h. Extracted with methylene chloride. After routine work up, silica gel chromatography(hexanes/EtOAc=2:1) afforded 156mg (87%) azide **118**.

*1-N-Acetyl-5(R)-[(Benzyloxymethyl)-3(R),4(R)-bis(benzyloxy)-2(S)-(hydroxymethyl)-N-methyl-pyrrollidine* ***(*119):** To the mixture of 104mg (220mmol) **118** in 10ml dry THF was added 118mg PPh3 (450mmol). The mixture was heated to 50°C for 4h. and concentrated under reduced pressure. The residue was dissolved in 8ml pyridine, under 0°C, 91ul Ac₂O was added to the reaction mixture. 12 hours later, 50ml water was added to the reaction mixture, and extracted with CH₂Cl₂. The organic layer was dried over MgSO₄ and concentrated under reduced pressure. Purified by silica gel chromatography (CH₂Cl₂/MeOH=60:1) to get **119** 91mg (87% yield from **118**).

*Compound* **104**: 34mg **19** was dissolved in 6ml AcOH/THF/H₂O (4:2:1), 10mg Pd-C (10%) was added to the solution. The reaction mixture was hydrogenated under the pressure of 50psi. After 1 day, the catalyst was filtered off, and the filtrate was concentrated and purified by silica gel chromatography (CHCl₃/MeOH/H₂O=6:4:0.7) to yield 16.6mg 4(89%). The spectra is same as before.

*2-O-acetyl-3-azido-diethyl-propanal* **121**: To a stirred suspension of 4.52g KHCO₃ in 150ml methanol was added 45ml acrolein diethyl acetal, 30ml benzonitrile, and 32ml 30% H₂O₂. The solution was warmed to 40°C in a water bath. After 8h, 10ml of H₂O₂ was added, after an additional 8h, 10ml more H₂O₂ was added. The solution was allowed to react for an additional 20h and then 39g NaN₃ was added. The pH was adjusted to 7.5 with 1M H₂SO₄ and the mixture was maintained at that pH by adding 1M H₂SO₄ with a peristaltic pump. The solution was warmed to 30°C for 14h. After the reaction finished, the methanol was removed under reduced pressure. After 100ml water was added, the solution was extracted with methylene chloride. The organic layer was washed with brine, dried over Na₂SO₄, and then evaporated to remove the solvent. Hexane was added to precipitate benzamide. The mixture was filtered and the filtrate was evaporated under reduced pressure. The residue was dissolved in 36.4ml pyridine and reacted with 34ml Ac₂O. After 3h at room temperature, 8ml methanol was added to quench the excess Ac₂O. 150ml ethyl acetate was added, and then washed with water, 1N HCl, saturated NaHCO₃, and brine and dried over Na₂SO₄. Filtered and concentrated. Distillation of the residue yielded 52.5g of **121** (76%).

*3-azido-1-ol-diethyl propanal* **122**: A solution of 2.31 g **121** in 100ml of phosphate buffer solution (0.05M, pH7.0) was mixed with 100mg PS-80 at room temperature with stirring. The pH was maintained at 7.0 with a peristatic pump by adding 0.25N NaOH and the degree of conversion was monitored by the consumption of base. After 49% conversion, the reaction mixture was extracted with EtOAc. After routine work up, Flash chromatography (EtOAc/hexanes=1:10) afforded 1.0g (45%) of the wanted enantiomer **122.**

*(R)-N-acetyl-2-diethoxymethylaziridine* **124:** To a 50ml flask containing 20ml anhydrous toluene and 1.0g **122** was added 2.62g triphenylphosphine. The solution was stirred at room temperature until bubbling of N₂ stopped. The mixture was then heated to 120°C for 3h. The solvent was removed under reduced pressure. Without further purification, the mixture was put to 30ml CH₂Cl₂ containing 10.0g of K₂CO₃. To this mixture was added 1.0ml of acetic anhydride. The mixture was stirred at room temperature for 15h. The mixture was filtered and the solvent was removed under reduced pressure. The residue was purified by silica gel chromatography to yield 0.280g of **124** (30% yield).

*(R)-3 Azido-2-acetamidopropanal Diethyl Acetal* **125:** A mixture containing 3.26g of **124** and 15g of sodium azide in 140ml of DMF was added 140ml of ZnCl₂ (1.0M solution in Et₂O), The mixture was stirred at 75°C for 3 days and then extracted with EtOAc. The organic layer was washed with water, dried over MgSO₄ and concentrated. The residue was purified by silica gel chromatography (hexane/EtOAc=3:2) to yield 2.52g of **125** (62%).

*Compound* **126**: A mixture containing 500mg of **125** and 15ml of HCl buffer (pH=1) was heated to 45°C for 10h. A solution of DHAP (1.7ml, 424mM) was then added, and the pH was adjusted to 6.5 with 1N NaOH. To this solution, rabbit muscle FDP aldolase (250 units) was added, and the mixture was stirred slowly at room temperature until DHAP was consumed completely based on enzymatic assay²⁰. The mixture was passed through AG-1(8 resin(HCO²⁻) ( 2.415cm) and eluted with water (200ml), 0.1M NaCl (150ml), 0.4M NaCl (300ml) successively. After adding 100ml of water to the fraction eluted by the 0.4M NaCl, the pH was adjusted to 4.7 with 1N HCl and 600 units acid phosphatase (from sweet potato, type X) was added, and the mixture was shaken slowly at 37°C for 1d. The pH was adjusted to 7.0, and water was removed by evaporation. The residue was treated with methanol. The soluble portion was collected and purified by silica gel chromatography (CHCl₃/MeOH/H₂O=8:2:0.1) to yield 34mg(55%) of **126.**

*1,2-Dideoxy-2-acetamidouojirimycin* **105:** To a solution of 30mg of **126** in 5 ml of EtOH was added 10mg of Pd-C, and the mixture was hydrogenated under the pressure of 50psi. After 1 day, the catalyst was filtered off, and the filtrate was concentrated and purified by silica gel chromatography (CHCl₃/MeOH/H₂O=6:4:0.7) to yield 21mg(87%) of **105.**

*N-Methyl-1,2-Dideoxy-2-acetamidonojirimycin* **106:** A solution containing 10mg of **105**, formaldehyde (300ul, 37%wt solution), and 10mg of 10% Pd-C was hydrogenated under 50psi of hydrogen in 5ml of methanol/water(1:1 solution) for 24h. The solvent was removed under reduced pressure. Silica gel chromatography (CHCl₃/MeOH/H₂O=6:4:0.7) afforded **106** 10mg (92%).

*2-acetamido-2 deoxy-3,4,6-tri-O-acetyl-thiozoline* **128:** A solution of 1.0g of 2-acetamido-2 deoxy-1,3,4,6-tetra-O-acetyl -β-D-glucopyranose **127** in 10ml of toluene was treated with 0.68g of Lawesson's reagent, and the reaction mixture was heated at 800C for 1.5h, cooled, and then concentrated. The residue was chromatographed on the silica gel chromatography (EtOAc/methylene chloride=3:7) to yield 0.89g (100%) of **128.**

*NAG-thioazoline* **107:** The thioazoline triacetate (0.22g) **128** dissolved in 3 ml methanol was treated with 70ul of 25% w/w methanolic sodium methoxide and the reaction mixture was stirred at room temperature for 30min. The reaction was neutralized to pH7.0 with H-from resin, and then concentrated to a solid residue. Extracted with methylene chloride, and the organic layer was concentrated. Flash chromatography(methylene chloride/methanol=4:1) afforded a white solid 117mg(85%) of the thioazoline **107.**

*Sulfated NAG-thiozoline* **108:** To a solution of 55mg **107** in the 4ml pyridine was added 35mg SO₃(NMe₃. The mixture was first stirred under 0°C for 2h, and then stayed at room temperature overnight. After concentration, silica gel chromatography (methylene:chloride/methanol=4:1) afforded 78mg(87%) **108.**

*Silyl ether **203***: To a 1.2ml DMF solution containing 70mg(0.32mmol) iminocyclitol **3** was added 84ul(0.48mmol) triethylamine and then cooled to 0°C, 72mg TBDMSCl(0.46mmol) was added and this mixture was stirred for overnight at room temperature. After the reaction finished, the solvent was removed under vacuum and the residue was purified by silica gel chromatography(CH₃Cl/MeOH=9:1) to afford the silyl ether **203** 93mg(88% yield).

*6-OH iminocyclitol **205***: A solution of 7mg **203**(0.02mmol) in 2ml pyridine was cooled down to 0°C and then 0.1ml Ac₂O was added. The mixture was stirred for 5.0hrs at room temperature. The solvent was then removed under vacuum and dried further under high vacuum. The crude silyl ether **204** residue was dissolved in a mixture solvent (AcOH/H₂O/THF=5: 1:3). The reaction mixture was stirred for overnight at 50°C. After the reaction was completed, the solvent was removed under vacuum and the residue was purified by silica gel chromatography (EtOAc/MeOH=4:1) to yield **205** 4.5 mg (75% from **203).**

*Sulfation of compound **205***: Under argon protection, 15mg SO₃(pyridine complex and 3mg(0.01mmol) compound **205** was dissolved in 0.5ml pyridine. The mixture was stirred for overnight at room temperature. After no starting material was left, the solvent was removed and the residue was purified by silica gel chromatography (EtOAc/MeOH=2:1) to afford 3.1mg **206** (82%).

*6-Sulfate α-iminocyclitols* **207**: 2mg(0.005mmol) protected 6-sulfate α-iminocyclitol **206** was dissolved in 2ml MeOH and then 2 drops of the 25% MeONa was added to the solution. The reaction was completed in 30 minutes and was quenched by acidic resin(H⁺). The solvent was then removed and the residue was purified by reverse phase column (Lichroprep RP-18) to afford 1.3 mg **207** (85%).

*6-Silyl ether protected* β-*intinocyclitol* **208**: 45mg(0.2mmol) β-iminocyclitol **202** and 60ul triethylamine(0.4mmol) were dissolved in 2ml DMF. The mixture was cooled down to 0°C. 71ul(0.3mmol) of TBDMSOTf was then added dropwise to the reaction system. The reaction was completed in 1.0hour. After removal of the solvent, the residue was purified by silica gel chromatography (CH₃Cl/MeOH=10:1) to afford 62mg **208** (90%).

*Benzylation of* **208:** 70mg(0.21mmol) of silyl ether **208** was dissolved in 3ml DMF under the protection of argon, the solution was then cooled to 0°C, 24mg 60% NaH(0.56mmol) was added. The mixture was stirred for 10 minutes before 101ul BnBr(0.46mmol) was added. The temperature was then increased to 25°C. The reaction was completed after 12 hours. The reaction mixture was then poured into 20ml ice-water. The resulted mixture was extracted with EtOAc. The organic phase was then dried over anhydrous Na₂SO₄ and concentrated. The residue was purified by silica gel chromatography (hexane/EtOAc=1:2) to afford 97mg, fully protected iminocyclitol **209** (90%).

*6-OH β-iminocyclitol* **210:** 25.8mg(0.05mmol) **209** was dissolved in 3ml THF. The mixture was cooled down to 0°C. 76ul TBAF solution (1.0M in THF) was added dropwise. The mixture was stirred for 4 hours at room temperature until the reaction was completed. The solvent was removed and the residue was purified by silica gel chromatography (MeOH/EtOAc=1:10) to give 16mg 10 in 80% yield.

*Sulfation of* **210:** 14mg **210** (0.035mmol) and 56mg SO₃ (pyridine (10 eqiv.) were dissolved in 2ml pyridine. The mixture was stirred for overnight at room temperature. After the reaction was completed, the solvent was removed and the residue was purified by silica gel chromatography (MeOH/EtOAc=1:4) to afford 13mg **211** (80%).

*6-Sulfate* β*-iminocyclitols* **212**: 20mg(0.042mmol) benzyl ether protected iminocyclitol **211** was dissolved in 3ml MeOH-H₂O(1:1) 30mg Pd(OH)/C was added. The mixture was hydrogenated under 1.0atm H₂ pressure for 12 hours. After the reaction was completed, the solvent was removed and the residue was purified by silica gel chromatography (CH₃Cl/MeOH/H₂O=6:4:0.7) to afford 9.3mg, **212** (75%).

*Methylation of 6-sulfated 3,4-diacetyl* α*-iminocyclitol* **206:** 5mg(0.013mmol) **206** was dissolved in 2ml MeOH and the mixture was stirred for 1.0 hour at 50°C until the reaction was completed. The solvent was removed and the residue was purified by silica gel chromatography (EtOAc/MeOH=2:1) to afford 4.6mg **215** (90%)

*6-Sulfate methyl ester α-iminocyclitols* **216:** 2mg(0.005mmol) protected 6-sulfate methyl ester **215** was dissolved in 2ml MeOH and then 2 drops of the 25% MeONa was added to the solution. The reaction was completed in 30 minutes and was quenched by acidic resin(H⁻). The solvent was then removed and the residue was purified by reverse phase column (Lichroprep⁽ RP-18) to afford 1.3mg **216** (80%).

### Enzymatic assay

*Materials*: The source of enzymes and substrates are as follows. α-glucosidase (EC 3. 2. 1. 20) from *Saccharomyces sp.* and β-glucosidase (EC 3. 2. 1. 21) from *Sweet almond*: Toyobo Co., Ltd. (Osaka, Japan); β-*N*-Acetylglucosaminidase (EC 3.2.1.30) from bovine kidny and β-*N*-acetylhexosaminidase A and P (EC 3.2.1.52) from human placenta: Sigma Chemical Co. (St. Louis, MO, USA); *p*-nitrophenyl-α-D-glucopyranoside and *p*-nitrophenyl-β-D-glucopyranoside; *p*-Nitrophenyl 2-*N*-acetyl-2-deoxy-β-D-glucopyranoside (*p*-nitrophenyl-*N*-acetyl-β-glucosaminide or *p*-NP-GlcNAc): Seikagaku Kogyo Co., Ltd (Tokyo, Japan); sodium acetate, sodium dihydrogenphosphate and sodium hydrogenphosphate: Nacalai Tesque. Inc. (Kyoto, Japan). Double deionized water was prepared from a Milli-Q system from Millipore Corp. (Milford, MA, USA). Millex-GV syringe filters (0.22 µm x 4 mm i.d.) were purchased from Nihon Millipore Ltd. (Yonezawa, Japan).

*Kinetic analysis of α-glucosidase*: To a 1-mL disposable cuvette was added 950 µL of 0.1 M phosphate buffer (pH 7.0) solution, 20 µL of inhibitor solution, and 20 µL of 20 mM *p*-nitrophenyl-α-D-glucopyranoside solution. The solution was well mixed and warmed at 37 °C for 5 min, then 20 µL of the enzyme solution in 10 mM phosphate buffer (pH 7.0) containing 0.2% of BSA was added. The reaction was monitored at 400 nm on Beckmann DU-70 spectrophotometer for 15 seconds, and the initial rate of hydrolysis was calculated. The same procedure was repeated with three other substrate concentrations. After the initial rates were accumulated, the corresponding Lineweaver-Burk plot at that inhibitor concentration was constructed.

*Kinetic analyssis of β-glucosidase*: To a 1-mL disposable cuvette was added 950 µL of 0.1 M acetate buffer (pH 5.0) solution, 20 µL of inhibitor solution, and 20 µL of 20 mM *p*-nitrophenyl-β-D-glucopyranoside solution. The solution was well mixed and warmed at 37 °C for 5 min, then 20 µL of the enzyme solution, which was dissolved in ice-cold Tris -HCl buffer (pH 7.8) and diluted with 10 mM phosphate buffer (pH 7.0) containing 0.2% of BSA, was added. The reaction was monitored at 400 nm on a Beckmann DU-70 spectrophotometer for 15 seconds, and the initial rate of hydrolysis was calculated. The same procedure was repeated with three other substrate concentrations. After the initial rates were accumulated, the corresponding Lineweaver-Burk plot at that inhibitor concentration was constructed.

### Capillary zone electrophoresis

*Condition of capillary zone electrophoresis*: Assays were performed on a Waters Quanta 4000E capillary electrophoresis system, which was equipped with a 53 cm x 75 µm fused i.d. silica capillary. Detection was carried out by on-column measurement of u.v. absorption at 405 nm at 7.5 cm from the cathode. The capillary used was pretreated or regenerated with 0.1 M KOH (2 min) and elution buffer before each injection. Samples were loaded by means of hydrostatic pressure at 10 cm height for 30 sec (*ca* 38.4 nl). Electrophoresis was performed at 20 kV using 50 mM sodium borate (pH 9.2 for β-*N*-Acetylglucosaminidase assays, pH 9.4 for β-glucosidase assays, pH 10.2 for α-glucosidase and β-*N*-acetylhexosaminidase assay) as electrolyte at a constant temperature of 37°C. Pherograms were recorded on Millennium 2010 system from Millipore Corp.

*Kinetic analysis of β-N-acetylglucosaminidase*: Incubations were performed in a total volume of 50 µL. Unless otherwise stated, reaction mixtures contained 25 mM citrate buffer (pH 4.4), various amount of *p*-NP-G1cNAc (0.5-2.0 mM) and various amount of inhibitors with 6.25 mU of β-*N*-acetylglucosaminidase. After preincubation for 10 min at 37°C, the reaction was started by the addition of β-*N*-acetylglucosaminidase and the reaction mixture was incubated for 10 min at 37°C. Then the reaction was terminated by addition of 100 µL of 50 mM sodium borate.

*Kinetic analysis of β-glucosidase*: The procedure is same as that described for the analysis of β-*N*-acetylglucosaminidase except for the pH of reaction mixtures (pH 5.5), the substrate *p*-NP-Glc (0.5-4.0 mM), and the enzyme β-glucosidase (12.8 mU ).

*Kinetic analysis of α-glucosidase*: The procedure is same as that described for the assay of β-Gnash except for the pH of the reaction mixture (phosphate buffer pH 7.0), the substrate [*p*-NP-α-Glc (0.2-1.1 mM)], and the enzyme [α-glucosidase (5mU)]. The termination of the reaction was carried out by addition of 50 µL of 200 mM Na₂CO₃.

*Kinetic analysis of β-N-acetylhexosaminidase from human placenta*: Incubations were performed in a total volume of 20 µL. Unless otherwise stated, reaction mixtures contained 100 mM citrate buffer (pH 4.5), various amount of *p*-NP-GlcNAc (0.1-1.1 mM) and various amount of inhibitors with 3.35 mU of β-*N*-acetylhexosaminidase A and 1.76 mU of β-*N*-acetylhexosaminidase P. After preincubation for 10 min at 37°C, the reaction was started by the addition of β-*N*-Acetylglucosaminidase and the reaction mixture was incubated for 15 min at 37°C. Then the reaction was terminated by addition of 20 µL of 0.2 M sodium carbonate.

## Claims

1. An inhibitor of hexoaminidase or glycosidase represented by the following structure: 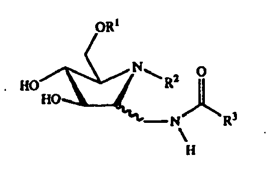 wherein:
R¹ is a sulfate group or a methyl sulfate group;
R² is selected from the group consisting of hydrogen, methyl, ethyl, and a branched or unbranched hydrocarbon having between 3 and 8 carbons; and
R³ is a hydrocarbon having between 1 and 20 carbon atoms.

2. An inhibitor according to claim 1 where R¹ is a sulfate group; R² is hydrogen, methyl, ethyl or any branched or unbranched hydrocarbon of between 3 and 8 carbon atoms; and R³ is a hydrocarbon group that has between 1 and 20 carbon atoms.

3. An inhibitor according to claim 2 where R³ is a hydrocarbon group possessing between 1 and 8 carbon atoms.

4. An inhibitor according to claim 2 where R³ is methyl.

5. An inhibitor according to claim 1 where R¹ is a methyl sulfate group; R² is selected from the group consisting of hydrogen, methyl, ethyl, and a branched or unbranched hydrocarbon of between 3 and 8 carbon atoms; and R³ is a hydrocarbon having between 1 and 20 carbon atoms.

6. An inhibitor according to claim 5 where R³ is a hydrocarbon having between 1 and 8 carbon atoms.

7. An inhibitor according to claim 6 where R³ is methyl.

8. An inhibitor of hexoaminidase or glycosidase represented by the following structure: 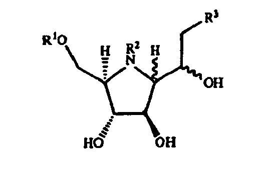 wherein:
R¹ is a sulphate group or a methyl sulphate group;
R² is selected from the group consisting of hydrogen, methyl, ethyl, and a branched or unbranched hydrocarbon having between 3 and 8 carbons; and
R³ is selected from the group consisting of hydroxyl and -NHC(O) R⁴, wherein R⁴ is a hydrocarbon having between 1 and 20 carbon atoms.

9. An inhibitor according to claim 8 wherein R¹ is sulfate; and R⁴ is a hydrocarbon having between 1 and 20 carbon atoms.

10. An inhibitor according to claim 9 wherein R⁴ is a hydrocarbon having between 1 and 8 carbon atoms.

11. An inhibitor according to claim 10 wherein R⁴ is methyl.

12. An inhibitor according to claim 8 wherein R¹ is methyl sulfate; and R⁴ is a hydrocarbon having between 1 and 20 carbon atoms.

13. An inhibitor according to claim 12 where R⁴ is a hydrocarbon having between 1 and 8 carbon atoms.

14. An inhibitor according to claim 13 where R⁴ is methyl.

15. A process for inhibiting a catalytic activity of a hexoaminidase or glycosidase *in vitro* comprising the step of contacting the hexoaminidase or glycosidase with an inhibitor selected from claims 1 to 14 of sufficient concentration for inhibiting said hexoaminidase or glycosidase.

16. The compounds of any one of claims 1 to 14 for use in therapy.

17. Use of a compound as claimed in any one of claims 1 to 14 for use in the manufacture of a medicament for treating arthritis.

## Patentansprüche

1. Hexoaminidase- oder Glykosidase-Inhibitor, der durch die folgende Struktur wiedergegeben wird: 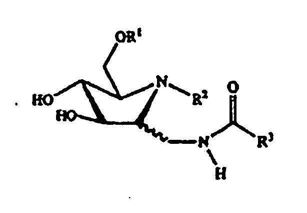 worin:
R¹ eine Sulfatgruppe oder eine Methylsulfatgruppe ist;
R² ausgewählt ist aus der Gruppe, bestehend aus Wasserstoff, Methyl, Ethyl und einem verzweigten oder unverzweigten Kohlenwasserstoff mit zwischen 3 und 8 Kohlenstoffen; und
R³ ein Kohlenwasserstoff mit zwischen 1 und 20 Kohlenstoffatomen ist.

2. Inhibitor nach Anspruch 1, wobei R¹ eine Sulfatgruppe ist; R² Wasserstoff, Methyl, Ethyl oder ein verzweigter oder unverzweigter Kohlenwasserstoff von zwischen 3 und 8 Kohlenstoffatomen ist; und R³ eine Kohlenwasserstoffgruppe ist, die zwischen 1 und 20 Kohlenstoffatomen aufweist.

3. Inhibitor nach Anspruch 2, wobei R³ eine Kohlenwasserstoffgruppe ist, die zwischen 1 und 8 Kohlenstoffatomen besitzt.

4. Inhibitor nach Anspruch 2, wobei R³ Methyl ist.

5. Inhibitor nach Anspruch 1, wobei R¹ eine Methylsulfatgruppe ist; R² ausgewählt ist aus der Gruppe, bestehend aus Wasserstoff, Methyl, Ethyl und einem verzweigten oder unverzweigten Kohlenwasserstoff mit zwischen 3 und 8 Kohlenstoffatomen; und R³ ein Kohlenwasserstoff ist, der zwischen 1 und 20 Kohlenstoffatomen aufweist.

6. Inhibitor nach Anspruch 5, wobei R³ ein Kohlenwasserstoff ist, der zwischen 1 und 8 Kohlenstoffatomen aufweist.

7. Inhibitor nach Anspruch 6, wobei R³ Methyl ist.

8. Hexoaminidase- oder Glykosidase-Inhibitor, der durch die folgende Struktur wiedergegeben wird: 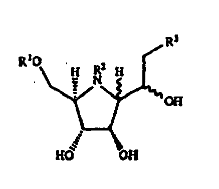 worin:
R¹ eine Sulfatgruppe oder eine Methylsulfatgruppe ist;
R² ausgewählt ist aus der Gruppe, bestehend aus Wasserstoff, Methyl, Ethyl und einem verzweigten oder unverzweigten Kohlenwasserstoff mit zwischen 3 und 8 Kohlenstoffen; und
R³ ausgewählt ist aus der Gruppe, bestehend aus Hydroxyl und -NHC(O)R⁴, worin R⁴ ein Kohlenwasserstoff mit zwischen 1 und 20 Kohlenstoffatomen ist.

9. Inhibitor nach Anspruch 8, wobei R¹ Sulfat ist; und R⁴ ein Kohlenwasserstoff ist, der zwischen 1 und 20 Kohlenstoffatomen aufweist.

10. Inhibitor nach Anspruch 9, wobei R⁴ ein Kohlenwasserstoff ist, der zwischen 1 und 8 Kohlenstoffatomen aufweist.

11. Inhibitor nach Anspruch 10, wobei R⁴ Methyl ist.

12. Inhibitor nach Anspruch 8, wobei R¹ Methylsulfat ist; und R⁴ ein Kohlenwasserstoff ist, der zwischen 1 und 20 Kohlenstoffatomen aufweist.

13. Inhibitor nach Anspruch 12, wobei R⁴ ein Kohlenwasserstoff ist, der zwischen 1 und 8 Kohlenstoffatomen aufweist.

14. Inhibitor nach Anspruch 13, wobei R⁴ Methyl ist.

15. Verfahren zum Inhibieren einer katalytischen Aktivität einer Hexoaminidase oder Glykosidase in vitro, umfassend den Schritt des Inkontaktbringens der Hexoaminidase oder Glykosidase mit einem Inhibitor, der aus Anspruch 1 bis 14 ausgewählt ist, mit ausreichender Konzentration zum Inhibieren der Hexoaminidase oder Glykosidase.

16. Verbindungen nach einem der Ansprüche 1 bis 14 zur therapeutischen Verwendung.

17. Verwendung einer Verbindung nach einem der Ansprüche 1 bis 14 zur Verwendung bei der Herstellung eines Medikaments zur Behandlung von Arthritis.

## Revendications

1. Inhibiteur de l'hexoaminidase ou de la glycosidase représenté par la structure suivante : 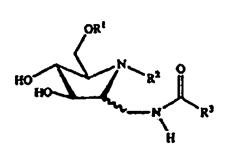 dans laquelle :
R¹ est un groupe sulfate ou un groupe méthylsulfate ;
R² est choisi dans le groupe constitué par un atome d'hydrogène, un groupe méthyle, éthyle et un hydrocarbure ramifié ou non ramifié comportant entre 3 et 8 atomes de carbone ; et
R³ est un hydrocarbure comportant entre 1 et 20 atomes de carbone.

2. Inhibiteur selon la revendication 1, dans lequel R¹ est un groupe sulfate ; R² est un atome d'hydrogène, un groupe méthyle, éthyle ou un quelconque hydrocarbure ramifié ou non ramifié comportant entre 3 et 8 atomes de carbone ; et R³ est un groupe hydrocarboné qui comporte entre 1 et 20 atomes de carbone.

3. Inhibiteur selon la revendication 2, dans lequel R³ est un groupe hydrocarboné comportant entre 1 et 8 atomes de carbone.

4. Inhibiteur selon la revendication 2, dans lequel R³ est un groupe méthyle.

5. Inhibiteur selon la revendication 1, dans lequel R¹ est un groupe méthylsulfate ; R² est choisi dans le groupe constitué par un atome d'hydrogène, un groupe méthyle, éthyle et un hydrocarbure ramifié ou non ramifié comportant entre 3 et 8 atomes de carbone ; et R³ est un hydrocarbure comportant entre 1 et 20 atomes de carbone.

6. Inhibiteur selon la revendication 5, dans lequel R³ est un hydrocarbure comportant entre 1 et 8 atomes de carbone.

7. Inhibiteur selon la revendication 6, dans lequel R³ est un groupe méthyle.

8. Inhibiteur de l'hexoaminidase ou de la glycosidase représenté par la structure suivante : 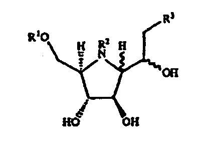 dans laquelle :
R¹ est un groupe sulfate ou un groupe méthylsulfate ;
R² est choisi dans le groupe constitué par un atome d'hydrogène, un groupe méthyle, éthyle et un hydrocarbure ramifié ou non ramifié comportant entre 3 et 8 atomes de carbone ; et
R³ est choisi dans le groupe constitué par un groupe hydroxyle et -NHC(O)R⁴, où R⁴ est un hydrocarbure comportant entre 1 et 20 atomes de carbone.

9. Inhibiteur selon la revendication 8, dans lequel R¹ est un groupe sulfate et R⁴ est un hydrocarbure comportant entre 1 et 20 atomes de carbone.

10. Inhibiteur selon la revendication 9, dans lequel R⁴ est un hydrocarbure comportant entre 1 et 8 atomes de carbone.

11. Inhibiteur selon la revendication 10, dans lequel R⁴ est un groupe méthyle.

12. Inhibiteur selon la revendication 8, dans lequel R¹ est un groupe méthylsulfate et R⁴ est un hydrocarbure comportant entre 1 et 20 atomes de carbone.

13. Inhibiteur selon la revendication 12, dans lequel R⁴ est un hydrocarbure comportant entre 1 et 8 atomes de carbone.

14. Inhibiteur selon la revendication 13, dans lequel R⁴ est un groupe méthyle.

15. Procédé d'inhibition d'une activité catalytique d'une hexoaminidase ou d'une glycosidase in vitro, comprenant l'étape de mise en contact de l'hexoaminidase ou de la glycosidase avec un inhibiteur choisi parmi les revendications 1 à 14 en une concentration suffisante pour inhiber ladite hexoaminidase ou ladite glycosidase.

16. Composés selon l'une quelconque des revendications 1 à 14 destinés à une utilisation en thérapie.

17. Utilisation d'un composé selon l'une quelconque des revendications 1 à 14 dans la fabrication d'un médicament pour traiter l'arthrite.
